# EUROPEAN PATENT APPLICATION

(11) **EP 4 609 904 A2**
(43) Date of publication of application: **03.09.2025**
(21) Application number: 25161160.4
(22) Date of filing: 28.02.2025
(51) Int. Cl.: A61M 25/00, A61M 25/06, A61M 39/12

(54) **EXPANDABLE INTRODUCER**

(30) Priority: 01.03.2024 US 202463560074 P; 26.07.2024 US 202463675889 P; 16.02.2025 US 202519054857
(71) Applicant: Medtronic, Inc., Minneapolis, Minnesota 55432 (US)
(72) Inventor: Mullen, Conleth A., Minneapolis, 55432 (US); Moran, Chris, Minneapolis, 55432 (US); Trotta, Antonia, Minneapolis, 55432 (US); Dunlea, Jake, Minneapolis, 55432 (US); Britton, Maeve, Minneapolis, 55432 (US); Farrell, Timothy Desmond, Minneapolis, 55432 (US); Heinze, Igor, Minneapolis, 55432 (US); Abuzaid, Adam, Minneapolis, 55432 (US); Mummoju, Karan, Minneapolis, 55432 (US); Lalor, Ian K., Minneapolis, 55432 (US); Anderson, Marc A., Minneapolis, 55432 (US); Kenny, Gavin, Minneapolis, 55432 (US); Mulvihill, Bernard Patrick, Minneapolis, 55432 (US); Huane, Daniel, Minneapolis, 55432 (US); Sail, Shridhar, Minneapolis, 55432 (US); O'Brien, Gary Eamonn, Minneapolis, 55432 (US); Mishra, Nikesh Kumar, Minneapolis, 55432 (US); Cullen, Catherine, Minneapolis, 55432 (US)
(74) Representative: Zimmermann & Partner Patentanwälte mbB

(57) **Abstract**

An expandable introducer includes an expandable introducer sheath coupled to an extending distally from an introducer hub via a connector. A distal portion of the introducer sheath includes a tip coupled to the sheath, the tip including a first circumferential portion and a second circumferential portion, the second circumferential portion being more flexible than the first circumferential portion. The second circumferential portion of the tip is circumferentially aligned with a folded portion of the sheath.

## Description

### CROSS-REFERENCE TO RELATED APPLICATIONS

The present application claims the benefit of U.S. Provisional Application No. 63/560,074, filed March 1, 2024, U.S. Provisional Application No. 63/675,889, filed July 26, 2024, and U.S. Patent Application No. 19/054,857, filed February 16, 2025, the contents of each of which are incorporated by reference herein in their entirety.

### FIELD OF THE INVENTION

The present invention relates to an expandable introducer device for providing percutaneous access to a patient's vasculature for a transcatheter device delivery system.

### BACKGROUND

An introducer device is used to provide percutaneous access to the vascular system of a patient and functions to permit the introduction and positioning of various minimally invasive delivery devices within the patient's vasculature. In general, making the smallest incision is the most desirable and leads to less complications, less trauma, and improved patient outcomes. Some delivery devices, however, are large and require large sheaths to accommodate and help deliver them to the desired site within the body.

### BRIEF SUMMARY OF THE INVENTION

In accordance with an example hereof, an introducer comprises: an introducer hub, the introducer hub including a proximal end, a distal end, and a central lumen extending from the proximal end to the distal end, the introducer hub including a distal tube at a distal portion of the introducer hub, the distal tube defining a portion of the central lumen; a sheath coupled to the introducer hub and extending distally therefrom; and a connector coupling the sheath to the distal tube of the introducer hub.

In accordance with another example hereof, in the introducer of any of the preceding or following examples: the distal tube comprises a cylinder having a stepped outer surface and including tube threads on a portion of the stepped outer surface; the connector comprises a cap comprising a cylinder having a stepped inner surface and including cap threads on a portion of the stepped inner surface; and the cap is configured to capture a proximal end of the sheath between the stepped inner surface of the cap and the stepped outer surface of the distal tube as the cap threads engage with the tube threads.

In accordance with another example hereof, in the introducer of any of the preceding or following examples: the distal tube further comprises a ratchet lock proximal of the tube threads, the ratchet lock comprising a plurality of teeth extending radially outwardly and circumferentially around the distal tube; and the cap further comprises at least one tooth extending radially inwardly proximal of the cap threads, wherein the at least one tooth is configured to engage the plurality of teeth of the ratchet lock to lock the cap to the distal tube.

In accordance with another example hereof, in the introducer of any of the preceding or following examples: the distal tube comprises a tube lip extending radially outwardly proximal of the tube threads; the cap includes a plurality of longitudinal legs extending proximally such that a proximal end of the legs is proximal of the cap threads, the plurality of longitudinal legs disposed adjacently around a circumference of the cap and including gaps between circumferentially adjacent longitudinal legs, the plurality of longitudinal legs comprising a cap lips extending radially inwards from the proximal end of the longitudinal legs; and the plurality of longitudinal legs are configured to flex radially outwardly for the cap lips to extend over the tube lip and are configured to flex back radially inwardly when the leg lips extend proximal of the tube lip such that the leg lips lock the cap to the distal tube.

In accordance with another example hereof, in the introducer of any of the preceding or following examples, the connector comprises a housing, a grab ring, and an O-ring.

In accordance with another example hereof, in the introducer of any of the preceding or following examples: the housing comprises a tube including distal lip extending radially inwardly from a distal end of the housing, a plurality of longitudinal legs at a proximal end of tube, the longitudinal legs separated circumferentially by a plurality of gaps between adjacent legs of plurality of longitudinal legs, and a proximal lip extending radially inward from a proximal end of the longitudinal legs; the distal tube includes a first groove extending radially inwardly from an outer surface of the distal tube and circumferentially around the distal tube and a second groove extending radially outwardly from an inner surface of the distal tube and circumferentially around the inner surface of the distal tube; and with a proximal end of the sheath disposed within the distal tube, the O-ring is disposed in the second groove of the distal tube between an inner surface of the distal tube and an outer surface of the sheath, the housing is configured to be translated over the sheath and over the distal tube proximally such that the longitudinal legs flex radially outwardly as the proximal lip extend over the distal tube until the proximal lip reaches the first groove such that the longitudinal legs flex back radially inwardly as the proximal lip is disposed within the first groove, and the distal lip of the housing extends radially inwardly distal of the distal end of the distal tube and abuts the outer surface of the sheath.

In accordance with another example hereof, in the introducer of any of the preceding or following examples, the grab ring is disposed between the distal lip of the housing and the distal end of the distal tube.

In accordance with another example hereof, in the introducer of any of the preceding or following examples: the connector comprises a locking clip configured to be disposed over the sheath, wherein the locking clip comprises a generally cylindrical tube including at least one clip arm proximal of a distal end of the locking clip, wherein the at least one clip arm is configured to flex radially inwardly and outwardly and includes a clip lip extending radially outwardly from the clip arm; the distal tube includes at least one slot extending from an outer surface to an inner surface thereof; the sheath includes a proximal lip extending radially outwardly therefrom; and with the locking clip disposed over the sheath such that a proximal end of the locking clip is adjacent the proximal lip of the sheath, the sheath and locking clip are configured to be inserted into the distal tube such that the at least one clip arm flexes radially inwardly until the clip lip aligns with the slot of the distal tube such the at least one clip arm flexes back radially outwardly and the clip lip extends into the slot to couple the sheath and the locking clip to the distal tube.

In accordance with another example hereof, in the introducer of any of the preceding or following examples, the locking clip further includes a distal lip extending radially outwardly from the distal end of the locking clip, wherein the distal lip is configured to abut a distal end of the distal tube when the clip lip extends into the slot.

In accordance with another example hereof, in the introducer of any of the preceding or following examples, the connector comprises a generally cylindrical tube including a distal portion overmolded over a proximal end of the sheath, a proximal portion configured to be disposed over the distal tube of the introducer hub, and band configured to be disposed around the proximal portion to couple the proximal portion to the distal tube of the introducer hub.

In accordance with another example hereof, in the introducer of any of the preceding or following examples, the distal tube of the introducer hub includes barbs extending radially outwardly along the distal tube, wherein the band of the connector is configured to deform the proximal portion of the generally cylindrical tube of the connector to couple the generally cylindrical tube of the connector to the distal tube of the introducer hub.

In accordance with another example hereof, in the introducer of any of the preceding or following examples, each barb extends circumferentially around the distal tube, and wherein each barb includes a barb distal end, a ramp extending radially outwardly and proximally from the barb distal end to a barb proximal end, and a shoulder at the barb proximal end extending radially inwardly form the barb proximal end.

In accordance with another example hereof, a system comprises: a dilator comprising a dilator hub and a dilator shaft coupled to and extending distally from the dilator hub, the dilator shaft including: a proximal portion having a first outer diameter; a middle portion having a second outer diameter; a proximal transition portion disposed between the proximal portion and the middle portion, the proximal transition portion tapering in a distal direction from the first outer diameter to the second outer diameter; and a distal portion extending distally from the middle portion, the distal portion including a distal cylindrical portion having a third outer diameter, wherein the first outer diameter is larger than the second outer diameter and the third outer diameter, and wherein the third outer diameter is larger than the second outer diameter.

In accordance with another example hereof, in the system of any of the preceding or following examples, the dilator shaft further includes a distal transition portion disposed between the middle portion and the distal cylindrical portion, wherein the distal transition portion flares outwardly distally from the second outer diameter of the middle portion to the third outer diameter of the distal cylindrical portion.

In accordance with another example hereof, in the system of any of the preceding or following examples, the distal transition portion flares linearly outwardly from a proximal end adjacent a distal end of the middle portion to a distal end of the distal transition portion adjacent a proximal end of the distal cylindrical portion.

In accordance with another example hereof, in the system of any of the preceding or following examples, the distal transition portion includes a proximal section and a distal section, the proximal section flaring radially outwardly with a curved concave profile, and a distal section flaring outwardly with a curved convex profile.

In accordance with another example hereof, in the system of any of the preceding or following examples, the distal transition portion includes a proximal section extending distally from the middle portion, a middle section extending distally from the proximal section, and a distal section distal extending distally from the middle section, wherein the proximal section flares outwardly in a distal direction, the middle section is generally cylindrical, and the distal section flares outwardly in a distal direction.

In accordance with another example hereof, in the system of any of the preceding or following examples, the proximal section and the distal section flare linearly outwardly.

In accordance with another example hereof, in the system of any of the preceding or following examples, each of the proximal section and the distal section includes curved concave profile and a curved convex profile.

In accordance with another example hereof, in the system of any of the preceding or following examples, the distal portion of the dilator shaft further includes a distal tapered tip extending distally from the distal cylindrical portion wherein the distal tapered tip includes an outer diameter than tapers distally.

In accordance with another example hereof, in the system of any of the preceding or following examples, the system further comprises an introducer comprising an introducer hub and an expandable sheath coupled to and extending distally from the introducer hub, wherein the expandable sheath is expandable from a radially compressed state wherein the expandable sheath has a first inner sheath diameter and a first outer sheath diameter to a radially expanded state wherein the sheath has a second inner sheath diameter larger than the first inner sheath diameter and a second outer sheath diameter larger than the first outer sheath diameter.

In accordance with another example hereof, in the system of any of the preceding or following examples: the first outer diameter of the dilator shaft proximal portion is sized to fit within the introducer hub; the second outer diameter of the dilator shaft middle portion is smaller than the first inner sheath diameter of the sheath; and the third outer diameter of the dilator shaft distal cylindrical portion is approximately equal to the first outer sheath diameter of the sheath.

**In** accordance with another example hereof, an expandable introducer sheath comprises: a shaft configured to be expanded from a radially unexpanded, folded state to a radially expanded, unfolded state in reaction to a device being extended through a central lumen of the expandable sheath, wherein the shaft includes: an inner liner; and an outer wall disposed radially outwardly of the inner liner, the outer wall having a variable thickness around a circumference of the shaft; and a tip coupled to a distal portion of the shaft, the tip including a first circumferential portion and a second circumferential portion, wherein the second circumferential portion is more flexible than the first circumferential such that the second circumferential portion is configured to radially expand when the in reaction to the device extending through tip.

In accordance with another example hereof, in the expandable introducer sheath of any of the preceding or following examples, in the radially expanded, unfolded state, the shaft includes a thick-walled region and a thin-walled region, wherein the thin-walled region is configured to fold when the shaft is in the radially unexpanded, folded state.

In accordance with another example hereof, in the expandable introducer sheath of any of the preceding or following examples, in the radially unexpanded, folded state, the shaft includes a folded region and an unfolded region, wherein the second circumferential portion is circumferentially aligned with the folded region.

In accordance with another example hereof, a method of forming a tip of an introducer sheath comprises: loading a shaft over a mandrel, the shaft including a liner, an inner layer, and an outer layer on a mandrel, the liner being disposed on the mandrel, the inner layer being disposed over the liner, and the outer layer being disposed over the inner layer, wherein the outer layer does not extend around the entire circumference of the inner layer, and wherein the liner, the inner layer, and the outer layer are not fused together; inserting a shim between the outer layer and the inner layer at a tip region of the shaft; fusing the liner, the inner layer, and the outer layer along the shaft except where the shim is disposed between the inner layer and the outer layer; removing the shim from between the inner layer and the outer layer, thereby leaving a gap between the inner layer and the outer layer in the distal region; folding the shaft to a radially unexpanded, folded state by folding a foldable region of the shaft where the outer layer does not extend around the circumference of the inner layer; shape setting the shaft to the radially unexpanded, folded state; removing an inner fold of the folded portion in the tip region; inserting a tip extrusion between the inner layer and the outer layer within the shaft in the radially unexpanded, folded shape, the tip extrusion including a first circumferential portion and a second circumferential portion, wherein the second circumferential portion is more flexible than the first circumferential portion, wherein the second circumferential portion is circumferentially aligned with the folded portion of the shaft; fusing the tip region of the shaft such that the first circumferential portion of the tip extrusion is fused to the inner layer and the outer layer, and the second circumferential portion of the tip extrusion is not fused to the inner layer and the outer layer.

In accordance with another example hereof, the method of any of the preceding or following examples further comprises, prior to fusing the tip region, inserting a tip extrusion shim under the second circumferential portion of the tip extrusion to prevent the second circumferential portion from fusing with the inner layer and the outer layer.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 shows a perspective side view of an expandable introducer according to embodiments hereof.
FIG. 2 is an exploded view of the expandable introducer of FIG. 1.
FIG. 3 shows a cross-section of a sheath of the expandable introducer of FIG. 1 in an expanded, unfolded state according to embodiments hereof.
FIG. 4 shows a cross-section of a sheath of the expandable introducer of FIG. 1 in an unexpanded, folded state according to embodiments hereof.
FIG. 5 shows a cross-section of a sheath of the expandable introducer of FIG. 1 in an expanded, unfolded state according to embodiments hereof.
FIG. 6 shows perspective view of a portion of a coil of the sheath of the expandable introducer of FIG. 1.
FIG. 7A shows a schematic illustration of a hybrid flexible tip for forming the tip of the expandable introducer of FIG. 1.
FIG. 7B shows a schematic cross-sectional view taken along line 7B-7B of FIG. 7A according to embodiment hereof.
FIG. 7C shows a schematic cross-sectional view taken along line 7B-7B of FIG. 7A according to another embodiment hereof.
FIGS. 8-14 show steps in a method of forming a tip of the expandable introducer of FIG. 1 according to embodiments hereof.
FIG. 15 shows a perspective distal end view of the tip of the expandable introducer of FIG. 1 according to embodiments hereof.
FIG. 16 shows a side view of the tip of the expandable introducer of FIG. 1 with a delivery catheter disposed through the tip according to embodiments hereof.
FIG. 17 shows an introducer hub of an introducer according to embodiments hereof.
FIG. 18 shows a cap of an introducer configured to couple an introducer sheath to the introducer hub of FIG. 17.
FIG. 19 shows an introducer with the cap of the FIG. 18 coupling an introducer sheath to the introducer hub of FIG. 17.
FIG. 20 shows a close-up view of a portion of the introducer hub of FIG. 17.
FIG. 21 shows longitudinal cross-sectional view of a sheath coupled to the introducer hub of FIG. 17 using the cap of FIG. 18.
FIGS. 22-25 show steps in a method of coupling an introducer sheath to the introducer hub of FIG. 17 using the cap of FIG. 18.
FIG. 26 shows a longitudinal cross-section of an introducer according to embodiments hereof.
FIG. 27 shows a close-up view of a proximal end of a cap of the introducer of FIG. 26 locking with an introducer hub of the introducer of FIG. 26.
FIGS. 28 and 29 show side views of steps in a method of coupling an introducer sheath of the introducer of FIG. 26 using the cap of FIG. 26.
FIG. 30 shows an O-ring of a connector for connecting a sheath to an introducer hub of an introducer according to embodiments hereof.
FIG. 31 shows a grab ring of a connector for connecting a sheath to an introducer hub of an introducer according to embodiments hereof.
FIG. 32 shows a housing of a connector for connecting a sheath to an introducer hub of an introducer according to embodiments hereof.
FIG. 33 shows the grab ring of FIG. 31 disposed within the housing of FIG. 32.
FIG. 34 shows a side view of an introducer with the connector of FIGS. 31-33 coupling a sheath of the introducer to an introducer hub of the introducer.
FIG. 35 is a longitudinal cross-sectional view of the introducer of FIG. 34 with the connector of FIGS. 31-33 coupling the sheath to the introducer hub.
FIG. 36 shows an exploded view of an introducer according to embodiments hereof.
FIG. 37 shows a side view of the introducer of FIG. 36 with a locking clip thereof disposed on a sheath thereof.
FIG. 38 shows the introducer of FIG. 36 with the locking clip thereof coupling the sheath to the introducer hub.
FIG. 39 shows a perspective view of an introducer according to embodiments hereof.
FIG. 40 shows a longitudinal cross-sectional view of the introducer of FIG. 39.
FIG. 41 shows a perspective longitudinal cross-sectional view of the introducer of FIG. 39.
FIG. 42A shows a schematic illustration of a dilator according to embodiments hereof.
FIG. 42B shows a close-up view of a distal portion of the dilator shaft of the dilator of FIG. 42A.
FIG. 43 shows the dilator of FIG. 42A and an expandable introducer according to embodiments hereof.
FIG. 44 shows a distal portion of the dilator shaft of the dilator of FIG. 42A disposed through an introducer sheath of an introducer.
FIG. 45 shows a cut-away view of an introducer hub, a hemostatic sleeve, an introducer sheath, and a portion of the dilator shaft of FIG. 42A.
FIG. 46 shows a distal portion of the dilator shaft of the dilator of FIG. 42A disposed through an introducer sheath of an introducer.
FIG. 47 shows a distal portion of the dilator shaft of the dilator of FIG. 42A disposed through an introducer sheath of an introducer.
FIGS. 48-50 show the dilator shaft of the dilator of FIG. 42A being retracted proximally through an expandable introducer sheath.
FIG. 51 depicts a side view of a distal transition portion of the dilator of FIG. 42B, according to embodiments hereof.
FIG. 52 depicts a partial side view illustration of a distal tip of a sheath and the distal transition portion of the dilator of FIG. 51 according to embodiments hereof.
FIG. 53 depicts a side view of a distal transition portion of the dilator of FIG. 42B according to embodiments hereof.
FIG. 54 depicts a partial side view illustration of a distal tip of a sheath and the distal transition portion of the dilator of FIG. 53 according to embodiments hereof.
FIG. 55 depicts a side view of a distal transition portion of the dilator of FIG. 42B according to embodiments hereof.
FIG. 56 depicts a partial side view illustration of a distal tip of a sheath and the distal transition portion of the dilator of FIG. 55 according to embodiments hereof.
FIG. 57 depicts a side view of a distal transition portion of the dilator of FIG. 42B according to embodiments hereof.
FIG. 58 depicts a partial side view illustration of a distal tip of a sheath and the distal transition portion of the dilator of FIG. 57 according to embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary, or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

The terms "proximal" and "distal" herein are used with reference to the clinician using the devices. Therefore, "proximal" and "proximally" mean in the direction toward the clinician, and "distal" and "distally" mean in the direction away from the clinician. In other words, "proximal" and "proximally" mean towards the hub end of the introducer and "distal" and "distally" means toward the tip end of the introducer.

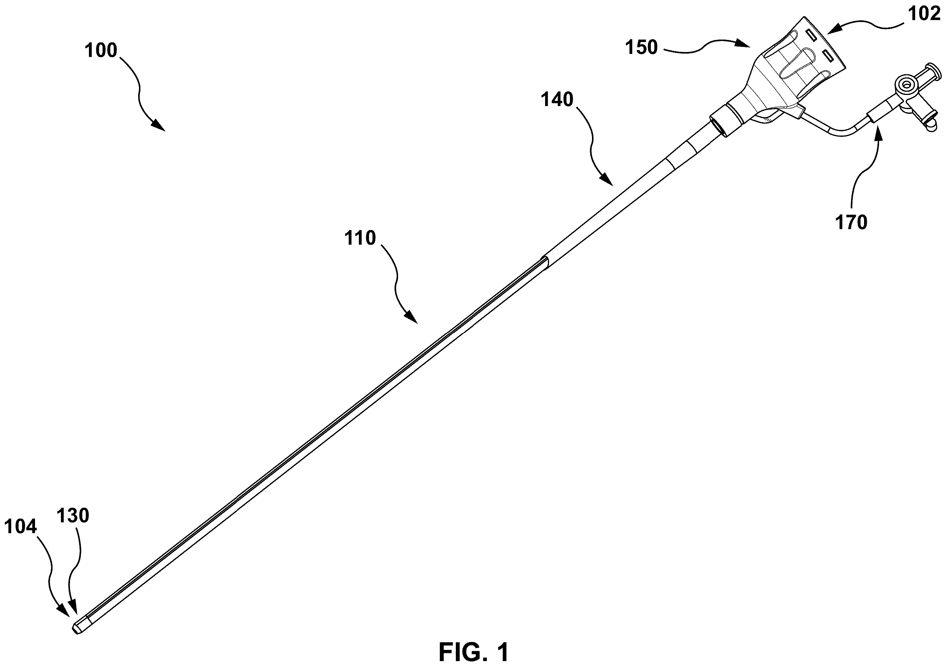

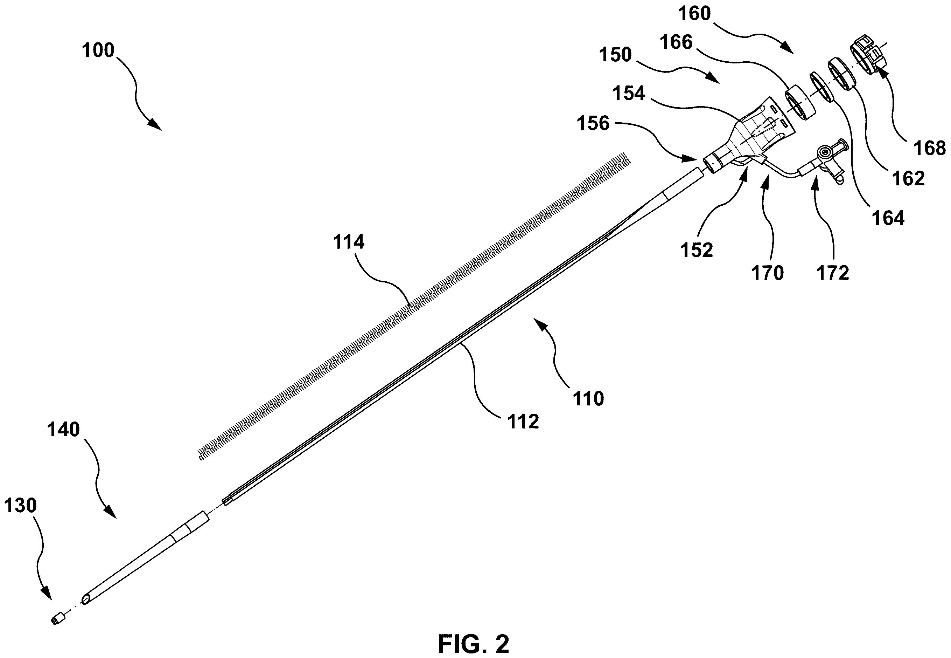

FIG. 1 shows an expandable introducer 100 according to embodiments herein. The expandable introducer 100 includes a proximal end 102 and a distal end 104. The expandable introducer 100 further includes an expandable sheath 110, a distal tip 130, a hemostatic sleeve 140, an introducer hub 150, and an extension tube 170. FIG. 2 shows an exploded view of the expandable introducer 100. As shown generally in FIG. 2, the expandable sheath 110 includes a shaft 112 and a coil 114, as described in more detail below. The distal tip 130 is coupled to and covers a distal end of the expandable sheath 110. A proximal end of the expandable sheath 110 is coupled to a distal end of the introducer hub 150. The hemostatic sleeve 140 is disposed over a proximal portion of the expandable sheath 110 and is coupled to the introducer hub 150. A proximal portion of the expandable sheath 110 is coupled to the introducer hub 150, as explained in more detail below.

The expandable introducer 100 may further include an extension tube 170 coupled to the introducer hub 150. In particular, a proximal end of the extension tube 170 may be coupled to a side port is coupled to a side port 152 of the introducer hub 150. The side port 152 is fluidly coupled to a passageway of the introducer hub 150, which is fluidly coupled to a central lumen of the sheath 110. Therefore, injecting saline, or other fluids, into the extension tube 170 results in the saline or other fluid being injected into the central lumen of the sheath 110. A stopcock 172 is coupled to the extension tube 170 and is configured to control the flow of saline, or other fluids, through the extension tube 170 and into the vasculature of the patient.

The introducer hub 150 of FIGS. 1 and 2 may be similar to the introducer hub described in International Patent Publication No. WO 2023/147422, which is incorporated by reference herein in its entirety. Thus, the introducer hub 150 generally includes a hub body 154, seal section 160, and a seal cap 168. The hub body 154 includes a lumen or passageway 156 extending from a proximal end to a distal end thereof. The seal section 160 may include a first or proximal seal 162, a second or middle seal 164, and a third or distal seal 166, as shown in FIG. 2. However, this is not meant to be limiting. For example, the seal section 160 may include exactly one seal, or exactly two seals, or exactly four seals, or four or more seals. In the embodiment shown, the first seal 162 is the proximal-most seal and the third seal 166 is the distal-most seal. The second seal 164 is disposed between, and is coupled to, the first seal 162 and the third seal 166. It should be understood that the first seal 162, the second seal 164, and the third seal 166 maintain hemostasis when various devices are inserted through the seals and when all devices are removed from the seals. The first seal 162 may also be referred to as a catheter seal, the second seal 164 may also be referred to as a slit seal, and the third seal 166 may also be referred to as a guidewire seal. The seal section 160 and the seals 162, 164, 166 may be similar to the seals described in International Patent Publication No. WO 2023/147422, which is incorporated by reference herein in its entirety. The seal cap 168 may be a generally cylindrically shaped piece of the introducer hub 150 that includes a body and a central lumen or passageway extending longitudinally therethrough. The seal cap 168 is configured to mate with the proximal end of the hub body 154 and abut the seal section 160 disposed within the hub body 154, maintaining compression in the seals and securing the seals in place. The seal cap 168 may be similar to the seal caps described in International Patent Publication No. WO 2023/147422, which is incorporated by reference herein in its entirety.

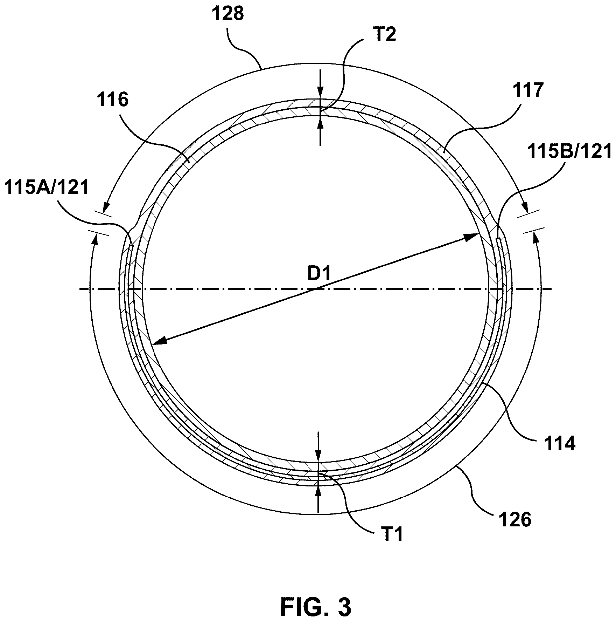

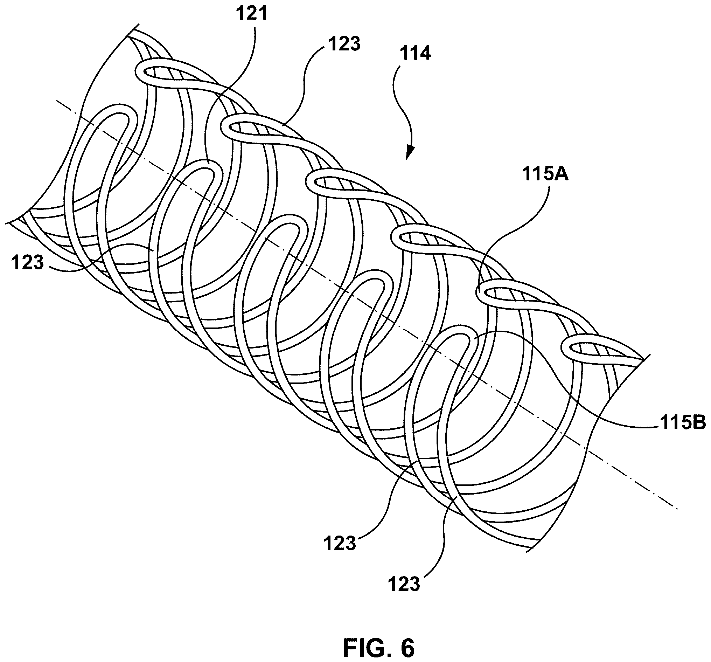

FIGS. 3-5 show cross-sectional views of the sheath 110 according to embodiments herein. FIGS. 3-5 show the sheath 110 in an unexpanded, folded state and the FIG. 6 show the sheath in an expanded, unfolded state. As shown in FIG. 2, the sheath 110 includes the shaft 112 and the coil 114. As shown in more detail in FIGS. 3-5, the sheath 110 includes a liner 116 and an outer wall 117 disposed radially outside and coupled to an outer surface of the liner 116. The coil 114 is embedded within the outer wall 117, as described in more detail below.

In embodiments, the liner 116 may be a thin, difficult to pierce, low-friction plastic tube disposed within and coupled to the interior surface of the outer wall 117. The liner 116 is configured to reduce friction forces and aid in the translation of devices through the central lumen of the sheath 110. In an embodiment, the liner 116 may have a thickness of about 0.0002 inch that is constant, or continuous, around the circumference of the sheath 110. The liner 116 may be polytetrafluoroethylene (PTFE), fluorinated ethylene propylene (FEP) or other materials known to those skilled in the art suitable for the purposes described herein, or any combination thereof.

In embodiments, the outer wall 117 is disposed outside of the liner 116 and may comprise polyether block amine (PEBAX) with a Shore D durometer value of about 63D to about 72D. In the embodiments shown, the outer wall 117 has a varying thickness around the circumference of the sheath 110, as described below.

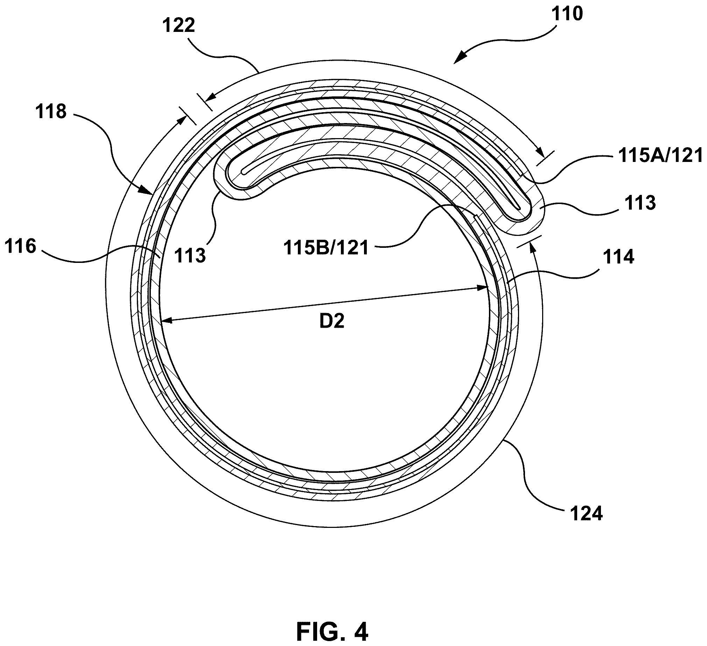

In embodiments, the coil 114 is formed of a wire comprising a shape memory material, such as nitinol. In embodiments, the wire has a diameter of about 0.008 inch. As shown in FIG. 3, in the radially expanded, unfolded state, the coil 114 extends partially around the circumference of the sheath 110. As can be seen in FIG. 4, in the radially unexpanded, folded state, the circumferential ends 115A, 115B of the coil 114 are closer to each other than in the radially expanded, unfolded state. The coil 114 is shape set to the radially unexpanded state shown in FIG. 3 such that after a device is passed through the sheath 110 to radially expand the sheath 110, the coil 114 will return the sheath 110 to the radially unexpanded, folded state of FIGS. 4. As shown in FIG. 6, the coil 114 may comprise a series of wrap or circumferential portions 123 and a series of crowns or bends 121, as described, for example, in U.S. Published Application Publication No. 2014/0236122 and/or U.S. Provisional Application No. 63/510,670, filed June 28, 2023, both of which are incorporated by reference herein in their entireties. In the embodiment of FIGS. 3-6, the circumferential ends 115A, 115B of the coil 114 do not overlap in the radially unexpanded, folded configuration, as shown in FIGS. 4 and 6. As can been seen in FIG. 6, the crowns or bends 121 define the circumferential ends 115A, 115B of the coil.

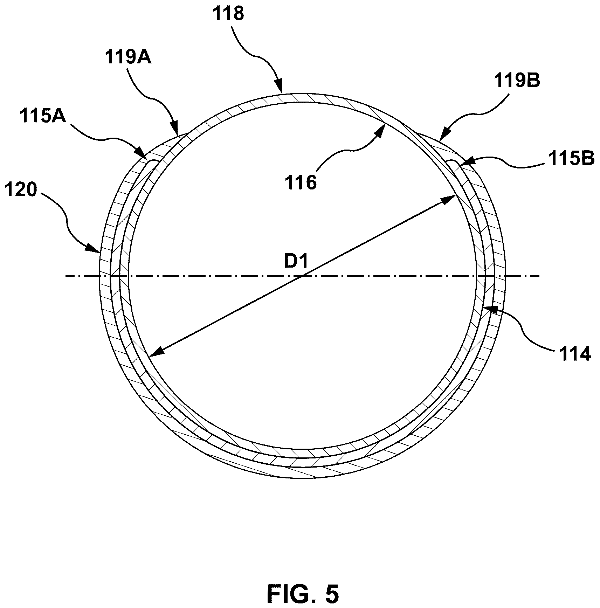

In the radially expanded, unfolded state, the sheath 110 may have an inner diameter D1, as shown in FIGS. 3 and 6. In embodiments, the inner diameter D1 may be in the range of about 17 French (about 5.67 mm) to about 22 French (about 7.33 mm). However, this is not meant to be limiting, and the inner diameter D1 may be any diameter suitable for the purposes described herein. In embodiments, the inner diameter D2 with the sheath 110 in the radially unexpanded, folded state shown in FIG. 5 may be in the range of about 10 French to about 16 French (about 3.33 mm to about 5.33 mm) depending on the application.

As shown in FIG. 4, the sheath 110 in the radially unexpanded, folded state includes a folded region 122 and a non-folded region 124. Also, in the radially expanded, unfolded state, the sheath 110 includes a coiled region 126 and a non-coiled region 128, as shown in FIG. 3, which may also be referred to as a thick-walled region 126 and a thin-walled region 128. In embodiments, the thick-walled region 126 has a first wall thickness T1 larger than a second wall thickness T2 of the thin-walled region 128. As shown in FIG. 3, the non-coiled or thin-walled region 128 includes the liner 116 and a thin portion of the outer wall 117. The coiled or thick-walled region 128 includes the liner 116, a thick portion of the outer wall 117, and the coil 114 embedded within the outer wall 117. Thus, the sheath 110 has variable wall thickness. In embodiments, the thickness of the outer layer 120 may taper to a thinner thickness as the sheath 110 transitions from the thick-walled region 126 to the thin-walled region 128. The second thickness T2 in the thin-walled region 128 may be about 0.0065 inch and the first thickness T1 in the thick-walled region 126 may be about 0.0225 inch. However, this is merely an example and is not meant to be limiting. The reduced wall thickness in the thin-walled/non-coiled region 128 assists in enabling the sheath 110 to fold into the radially unexpanded, folded state shown in FIG. 4.

As can be seen in FIG. 4, in the radially unexpanded, folded state, the coil 114 extends into the folded region 122 such that the bends/crowns 121 (circumferential ends 115A/115B) of the coil 114 are disposed adjacent to each other. As can been seen in FIG. 3, in the radially expanded, unfolded state, the bends/crown 121 (circumferential ends 115A/115B) of the coil 114 are spaced from each other, thereby forming the uncoiled region 128, or a gap, between the circumferential ends 115A, 115B of the coil 114. In an embodiment, the coil 114 extends greater than 50% around the circumference of the sheath 110 in the radially expanded, unfolded state, as shown in FIG. 3.

The sheath 110 is configured to radially expand from the radially unexpanded, folded state to the radially expanded, unfolded state, and radially compress back to the radially unexpanded, folded state. The sheath 110 maintains the radially unexpanded, folded state until a device is advanced through the sheath 110, during which the sheath 110 is configured to expand to the radially expanded, unfolded state. When the sheath 110 is in the radially unexpanded, folded state, the sheath 110 includes the folded region 122 and the non-folded region 124, as shown in FIG. 4. The folded region 122 is defined as the region where a section of the sheath 110 is folded over, as can be seen in FIG. 4. In other words, a portion of the sheath 110 is folded and bent to overlap with itself. The non-folded region 124 of the sheath 110 is defined as the remainder of the sheath 110 that does not include a fold. As can be seen in FIG. 4, when folded, the sheath 110 includes two folds or bends 113.

The liner 116 and the outer wall 117 may be formed by co-extrusion or a layered reflow lamination processes. In particular, in an embodiment, as shown in FIG. 5, the outer wall 117 may be formed of two layers of the same material. In particular, an inner layer 118 and an outer layer 120 of the same material may form the outer wall 117. In an embodiment, prior to forming the sheath 110, as shown in FIG. 5, the inner layer 118 is continuous around the entire circumference of the liner 116 and the outer layer 120 is not continuous and extends only partially around the circumference of the inner layer 118. Further, prior to forming the sheath 110, the coil 114 is disposed between the inner layer 118 and the outer layer 120. In an embodiment, the inner layer 118 may have thickness of about 0.0045 inch and the outer layer 120 may have a thickness of about 0.0016 inch. In such an embodiment, both inner layer 118 and the outer layer may comprise polyether block amine (PEBAX) with a Shore D durometer value of about 63D to about 72D. In the embodiments, the inner layer 118 and the outer layer 120 are formed from the same material having the same durometer. In particular, in an embodiment, the inner layer 118 and the outer layer 120 may both be formed of PEBAX with the same Shore D durometer and the durometer being about 63D. Thus, upon reflow, the inner layer 118 and the outer layer 120 meld together to form a single outer wall 117 with the coil 114 embedded therein.

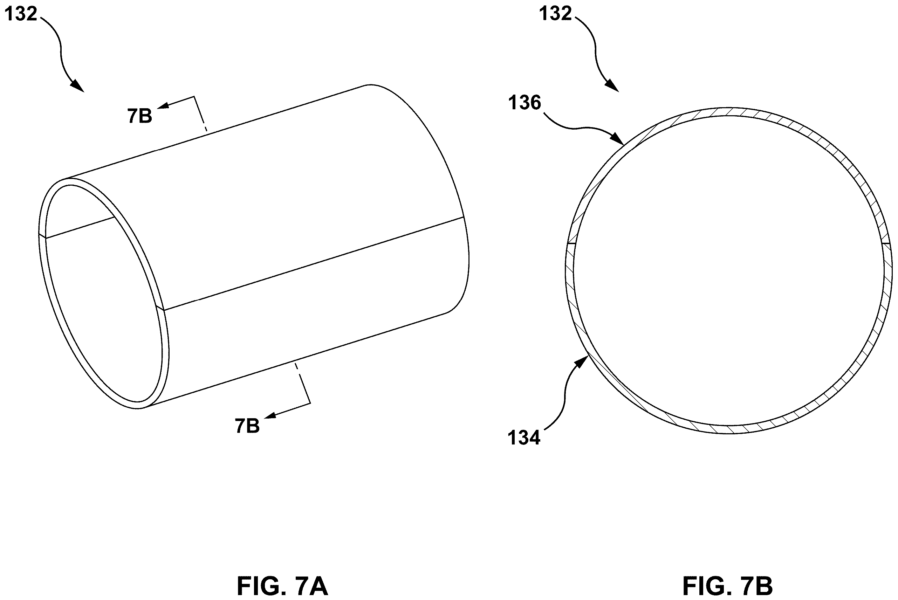

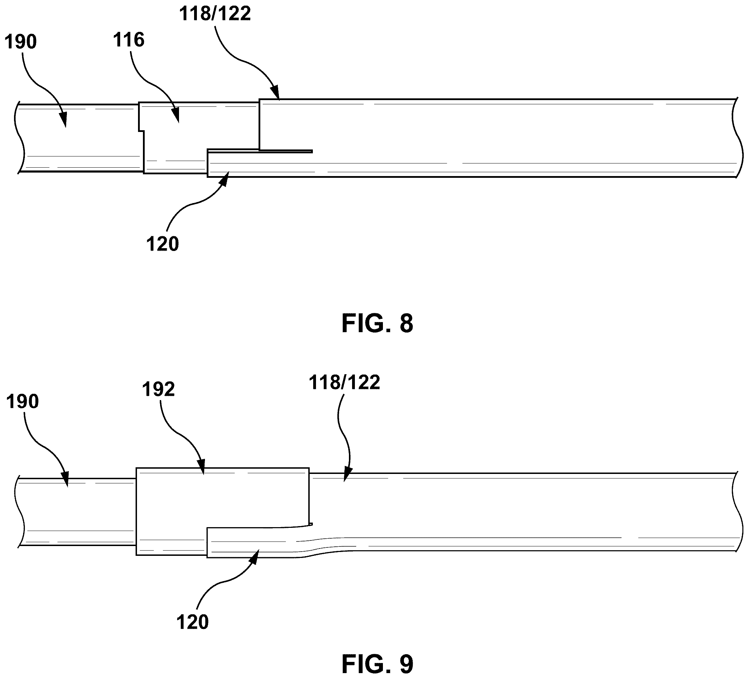

As briefly explained above, the tip 130 is coupled to and covers a distal end of the sheath 110. FIGS. 7A and 7B show a tip extrusion 132 and FIGS. 8-14 show a method of forming the tip 130 according to embodiments hereof. The coil 114 does not extend to the distal end of the sheath 110. Accordingly, there is a small length of the sheath 110 at the distal end thereof that does not include the coil 114. This distal end of the sheath 110 without the coil 114 is coupled to the tip extrusion 132 to form the tip 130.

The tip extrusion 132 is shown in FIGS. 7A-7C. As shown in FIG. 7A, the tip extrusion 132 is a generally cylindrical tube. In an embodiments, the tip extrusion has a length of about 6 mm to about 9 mm. FIG. 7B is a cross-section view of the tip extrusion 132 taken along line 7B-7B of FIG. 7A. The tip extrusion 132 includes a first circumferential portion 134 made of a first material and a second circumferential portion 136 made of a second material. The first material may be stiffer than the second material. The second material is flexible such that the second circumferential portion 136 is flexible and can expand with the folded portion of the sheath 110 when the sheath 110 radially expands. Thus, the first circumferential portion 134 is relatively stiff to aid in pushability of the distal tip 130 during insertion into a vessel, and the second circumferential portion 136 is relatively flexible to enable the distal tip 130 to conform to the profile of the inserted device, *i.e.,* radially expand to accommodate the inserted device. In an embodiment, the first material is PEBAX/polyamide, and the second material is polyurethane/soft durometer PEBAX.

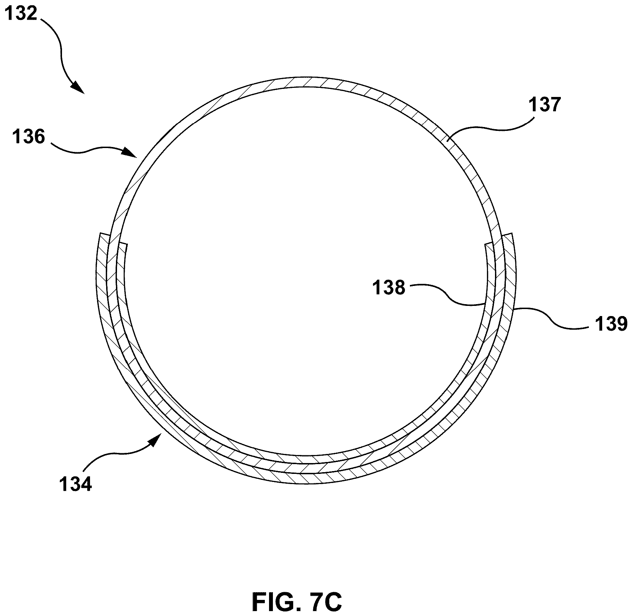

FIG. 7C shows another embodiment of a cross-section of the tip extrusion 132. In the embodiment of FIG. 7C the tip extrusion 132 includes three layers 137, 138, 139. In the embodiment shown, a first layer 137 extends 360 degrees. Further, the first layer 137 is flexible. The second layer 138 and the third layer 139 sandwich the first layer 137 and extend only partially around the inner and outer circumference thereof, respectively. Thus, the portion of the circumference with the first, second, and third layers 137, 138, 139 forms the first circumferential portion 134, and the portion of the circumference with only the first layer 137 forms the second circumferential portion 136. In an embodiment, the first layer 137 may be polyurethane, soft durometer PEBAX and the second and third layers 138, 139 may be PEBAX, polyamide. In other words, the first layer 137 is a soft, flexible material and the second and third layers 138, 139 are stiffer materials relative to the first layer 137. Further, although shown as layers, those skilled in the art will recognize that the layers are fused to form a single wall. Further, although shown with three layers with the middle layer being the flexible layer, those skilled in the art will recognize that other embodiments may be used, such as, but not limited to, two layers, or four or more layers, and that the flexible layer need not be the middle layer.

In a step of the method, the liner 116, the inner layer 118, and the outer layer 120 are loading onto a straight mandrel 190, as shown in FIG. 8. The liner 116, the inner layer 118, and the outer layer 120 are not bonded together at this stage of the method. As explained above, the outer layer 120 does not extend around the entire circumference of the sheath 110. Therefore, in FIG. 7, a portion of the circumference of the sheath 110, *i.e.,* the thin-walled region 126, does not include the outer layer 120 such that the inner layer 118 is the outermost layer of the thin-walled region 126.

In another step of the method, a shim 192 is inserted between the outer layer 120 and the inner layer 118, *i.e.,* under the outer layer 120 and over the inner layer 118, as shown in FIG. 9. The shim 192 may be a cylindrical tube approximately 6 mm in length. As shown in FIG. 9, only a portion of the circumference of the shim 192 is shown because a portion of the circumference of the shim 192 is disposed under the outer layer 120. The portion of the circumference of the shim 192 that is shown in FIG. 9 is aligned with the thin-walled region 126 of the circumference of the sheath 110. Because the thin-walled region 126 does not include an outer layer 120, the shim 192 is not hidden under the outer layer 120 in the thin-walled region 126 in FIG. 9.

With the shim 192 inserted between the inner layer 118 and the outer layer 120, a heat shrink wrap is loaded over the assembly. Heat and pressure are applied to fuse the layers together except in the area of the shim 192. The shim 192 may be formed of a material such as PTFE. During this step, the inner layer 118 (PEBAX) fuses to the liner 116 (PTFE) because the liner 116 is etched to allow for adherence. The fuse may be described as thermal tack rather than a weld, but it provides sufficient adherence for the device to function as desired. The shim 192 (PTFE) is not etched and therefore does not adhere to the inner layer 118 (PEBAX) or the outer layer 120 (PEBAX), and prevents fusion of the inner layer 119 to the outer layer 120.

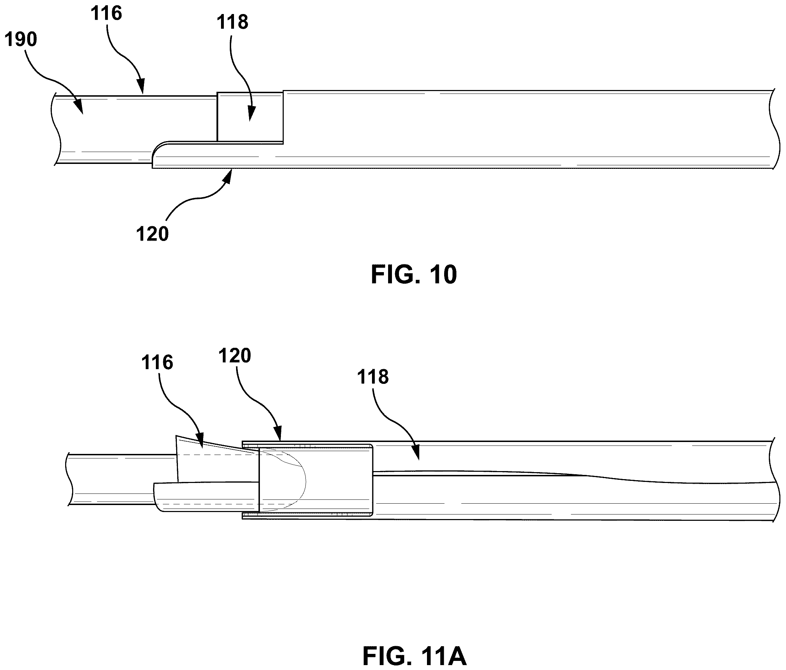

The shim 192 is then removed from the under the outer layer 120, as shown in FIG. 10, thereby leaving a gap between the inner layer 118 and the outer layer 120 at the distal end of the sheath 110 (i.e., the gap is equal to the length of the shim 192 inserted between the inner layer 118 and the outer layer 120). The sheath 110 is then folded to the radially unexpanded, folded state. A heat shrink wrap is loaded over the full length of the sheath 110. Heat and pressure are applied to set the shape of the sheath 110 in the radially unexpanded, folded state and the unfused layers are fused.

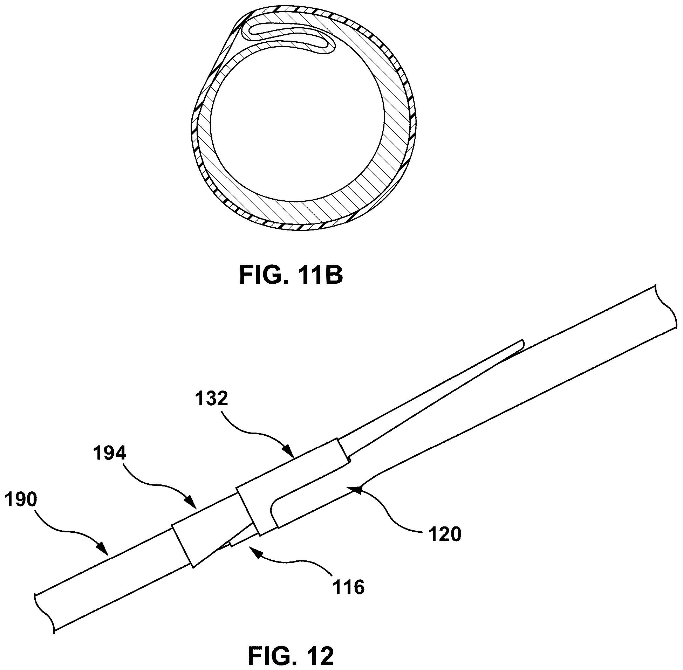

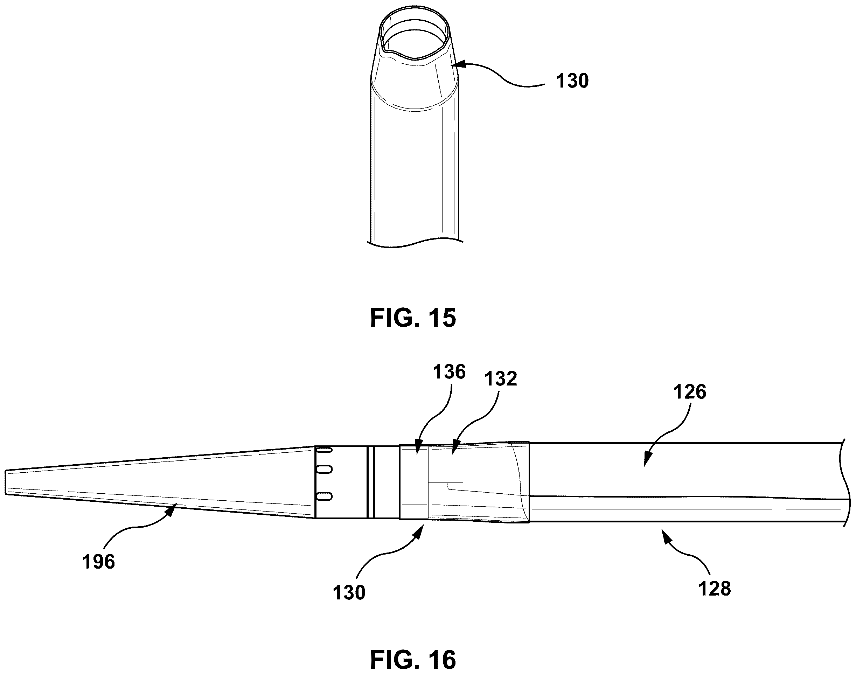

In another step, the inner fold 198 of the folded portion is cut away for a short distance from the distal end, as shown in FIG. 11B. In an example, about 3 mm of the distal end of the inner fold 198 of the folder portion is cut away. Due to this cut, after reflow, the distal profile of the tip will be circular, as shown in FIG. 15. The tip with the inner fold 198 cut away is shown in FIG. 11A.

In another step, the tip extrusion 132 is loaded under the outer layer 120 and over the inner layer 118, as shown in FIG. 12. The second (flexible) circumferential portion 136 of the tip extrusion 132 is circumferentially aligned with the folded portion of the sheath 110. In other words, the second (flexible) circumferential portion 136 of the tip extrusion 132 is circumferentially aligned between the circumferential ends 119A, 119B of the outer layer 120. Thus, the first (stiff) circumferential portion 134 of the tip extrusion 132 is circumferentially aligned with the outer layer 120. Further, a shim 194 is inserted under the second (flexible) circumferential portion 136 of the tip extrusion 132 and over the liner 116, as shown in FIG. 12. As can be seen in FIG. 12, when the shim 194 is inserted under the tip extrusion 132, the shim 194 forms an arc or partial cylinder shape. In practice, the shim 194 may be relatively flat and may bend to conform to the shape of the second (flexible) circumferential portion 136 of the tip extrusion 132, as shown in FIG. 12. The shim 194 prevents the second (flexible) circumferential portion 136 of the tip extrusion 132 from bonding to other layers during fuse in the folded region 122 of the sheath 110.

In another step of the method, a heat shrink wrap is loaded over the tip assembly. Heat and pressure are applied to fuse the materials of the tip assembly together, except where the shim 194 is located. Thus, during fusing, the tip extrusion 132 is fused to inner and outer layers 118, 120 in the non-folded region 124 of the sheath 110. The shim 194 prevents the tip extrusion 132 from bonding to the inner and outer layers 118, 120 in the folded region 122 of the sheath 110. After fusing, the shim 194 is removed and excess liner 116 is trimmed.

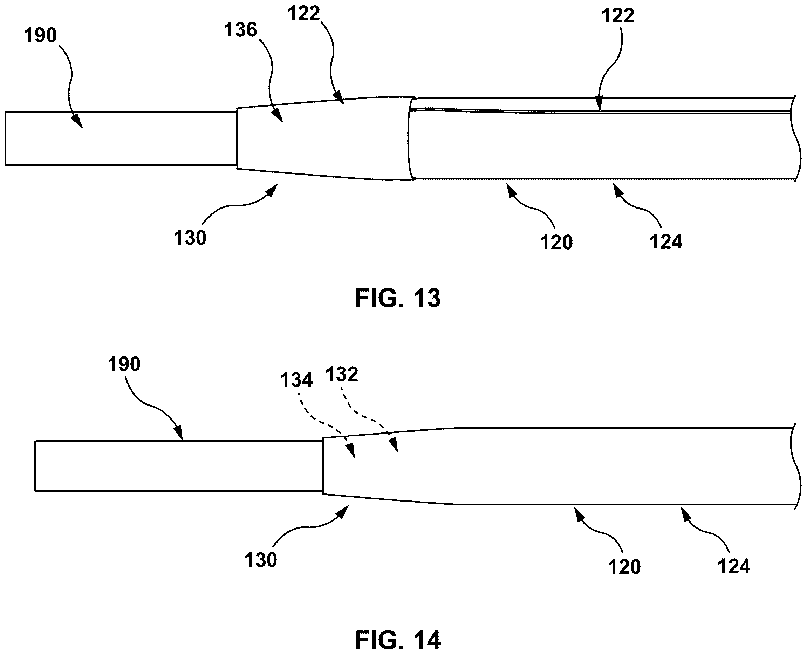

Upon completion of the method, the second (flexible) circumferential portion 136 of the tip extrusion 132 is circumferentially aligned with the folded region 122 of the sheath 110 and the first (stiff) circumferential portion 134 of the tip extrusion 132 is circumferentially aligned with the non-folded region 124 of the sheath 110. FIG. 13 shows the second (flexible) circumferential portion 136 and FIG. 14 shows the first (stiff) circumferential portion 134. As explained above, FIG. 15 shows the circular distal profile of the tip 130. FIG. 16 shows a delivery catheter 196 inserted through the distal tip 130, showing the second (flexible) circumferential portion 136 of the tip extrusion 132 radially expanding to accommodate the delivery catheter 196.

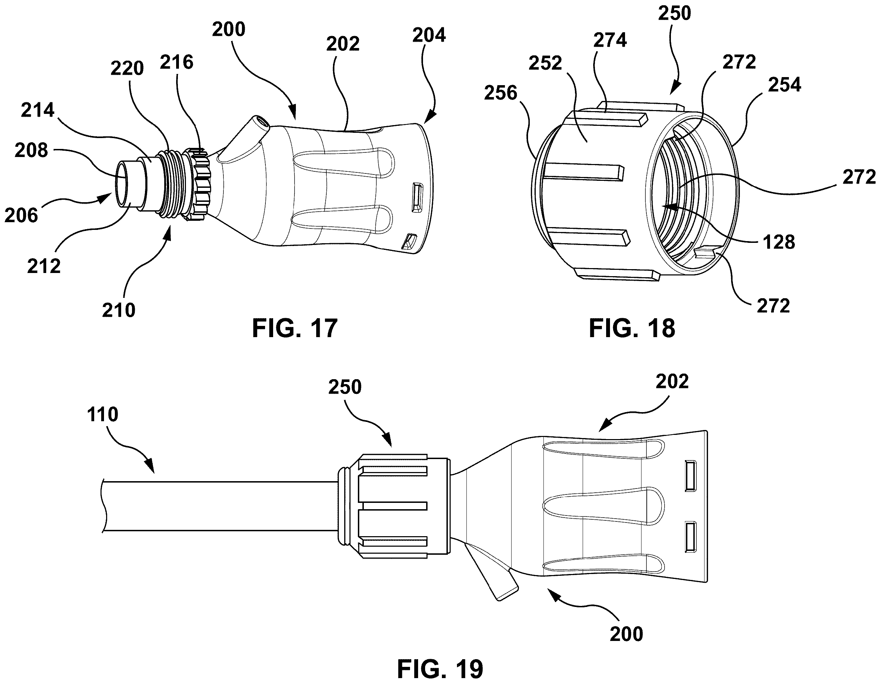

FIGS. 17-25 show devices and methods for securing a sheath of an introducer to an introducer hub 200 of the introducer. In embodiments, the sheath may be the sheath 110 as described above or may be other sheaths. In embodiments, the introducer hub 200 may be similar to the introducer hub 150 described above except for details described herein with respect to the introducer hub 200. However, this is not meant to be limiting, and the introducer hub 200 is not limited to the details described above with respect to the introducer hub 150.

FIG. 17 shows the introducer hub 200 according to embodiments herein. Those skilled in the art will recognize that all the details of the introducer hub 200 are not described and that other features may be included with the introducer hub 200 or that features may be excluded or modified. The introducer hub 200 includes a body 202 having a proximal end 204, a distal end 206, and a central lumen or passageway 208 extending from the proximal end 204 to the distal end 206. The introducer hub 200 may include seals, seal caps, and other features as described above or as known to those skilled in the art. The introducer hub 200 further includes a cap 250 for coupling the sheath 110 to the hub body 202.

In the embodiment shown, the body 202 of the introducer hub 200 includes a distal tube 210 extending proximally from the distal end 206 of the body. The distal tube 210 includes a first portion 212, a second portion 214 proximal of the first portion 212, and a ratchet lock 216 proximal of the second portion 214. In particular, as shown in FIGS. 17, 20, and 21, the first portion 212 is a cylindrical tube with an inner surface thereof defining a portion of the central passageway 208. The wall of the first portion has a first wall thickness such that an outer surface thereof defines a first outer diameter OD1. The second portion 214 extends proximally from the first portion 212. The second portion 214 is a cylindrical tube defining a portion of the central passageway 208. The wall of the second portion 214 has a second wall thickness thicker than the first wall thickness such that an outer surface of the wall of the second portion defines a second outer diameter OD2 larger than the first outer diameter OD1. The change in wall thickness from the first portion 212 to the second portion 214 creates a shoulder 218 between the first portion 212 and the second portion 214. The second portion 214 further includes threads 220 along a portion thereof.

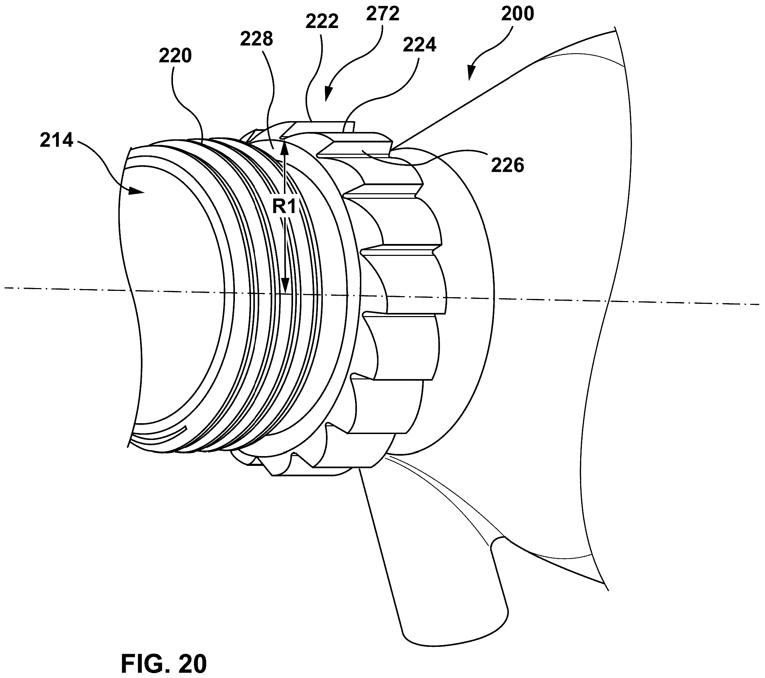

The ratchet lock 216 is disposed proximal of the second portion 214. The ratchet lock 216 includes a plurality of teeth 222 extending circumferentially. In particular, as best shown in FIG. 20 each tooth 222 includes a ramp 224 and a shoulder 226. Each ramp 224 extends radially outwardly and circumferentially from a first end spaced a first radius R1 from a central longitudinal axis CLA of the passageway 208 to a second end spaced a second radius R2 from the central longitudinal axis CLA of the passageway 208. The shoulder 226 extends from the second end substantially radially inward toward the central longitudinal axis CLA. The ratchet lock 216 may further include a shoulder 228 extending radially outwardly from the second portion 214, as shown in FIG. 20.

The cap 250 of the introducer hub 200 is configured to couple the sheath 110 to the hub body 202, as described in more detail below. In the embodiment shown, the cap 250 includes a cap body 252 including a proximal end 254, a distal end 256, and a central lumen 258 extending from the proximal end 254 to the distal end 256. The cap 250 is a substantially cylindrical, stepped tube. Thus, in the embodiment shown, the cap body 252 includes a first longitudinal portion 260, a second longitudinal portion 262 proximal of the first longitudinal portion 260, a third longitudinal portion 264 proximal of the second longitudinal portion 262, and a fourth longitudinal portion 266 proximal of the third longitudinal portion 264. The first, second, third, and fourth longitudinal portions 260, 262, 264, 266 are defined by having different internal diameters. Thus, the first longitudinal portion 260 has an inner surface defining a portion of the central lumen 258 and having a first inner diameter ID1, the second longitudinal portion 262 has an inner surface defining a portion of the central lumen 258 and having a second inner diameter ID2, the third longitudinal portion 264 has an inner surface defining a portion of the central lumen 258 and having a third inner diameter ID3, and the fourth longitudinal portion 266 has an inner surface defining a portion of the central lumen 258 and having a fourth inner diameter ID4. In the embodiment shown, the first inner diameter ID1 is smaller than the second inner diameter ID2, which is smaller than the third inner diameter ID3, which is smaller than the fourth inner diameter ID4. Further, a first shoulder 261 is disposed at the inner surface between the first longitudinal portion 260 and the second longitudinal portion 262, a second shoulder 263 is disposed at the inner surface between the second longitudinal portion 262 and the third longitudinal portion 264, and a third shoulder 265 is disposed at the inner surface between the third longitudinal portion 264 and the fourth longitudinal portion 266.

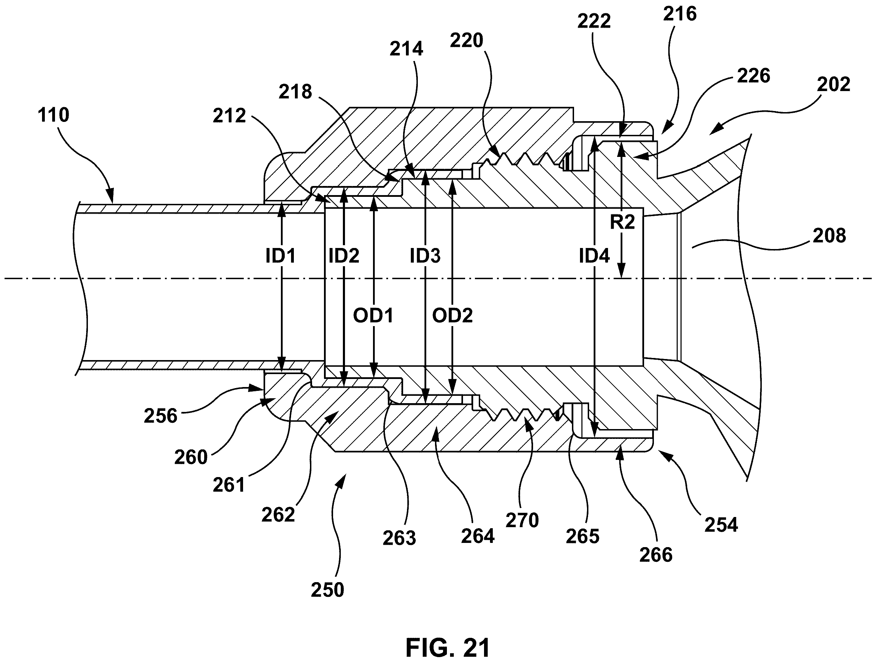

As explained in more detail below, the cap 250 is configured to capture a proximal end of the sheath 110 between the cap body 252 and the hub body 202. Thus, in the embodiment shown, the first inner diameter ID1 of the cap body 252 is smaller than the first outer diameter OD1 of the hub body 202, the second inner diameter ID2 of the cap body 252 is larger than the first outer diameter OD1 of the hub body 202 but smaller than the second outer diameter OD2 of the hub body 202, the third inner diameter ID3 of the cap body 252 is larger than the second outer diameter of the hub body 202 but smaller than an outer diameter of the hub body 202 at the ratchet lock 216, and the fourth inner diameter ID4 of the cap body is larger than the outer diameter of the hub body 202 at the ratchet lock 216, as shown in FIG. 21.

Further, the inner surface of the cap body 252 at the third longitudinal portion 264 includes inner threads 270 configured to mate with the threads 220 on the outer surface of the second portion 214 of the hub body 202. The inner surface of the cap body 252 at the fourth longitudinal portion 266 includes two teeth 272 (see FIG. 18) configured to lock onto the teeth 222 of the ratchet lock 216 of the hub body 202. Although the particular embodiment shows two teeth 272, this is not meant to be limiting, and more or fewer teeth 272 may be utilized in keeping with the objects of the present description. As shown in FIG. 18, the outer surface of the cap body 252 may include ridges 274 or other similar features to assist a user in gripping the cap 250 to advance the cap 250 proximally over the proximal portion of the sheath 110 and the distal portion of the hub body 202, as described below.

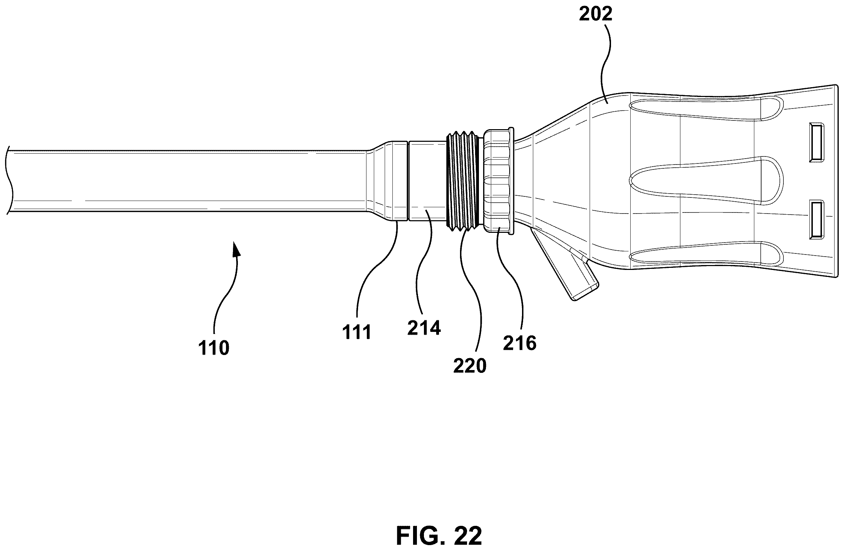

With reference to FIGS. 22-25, a method of coupling the sheath 110 to the introducer hub 200 will be described. In a step of the method, a flared proximal portion 111 of the sheath 110 is placed over the first portion 212 of the hub body 202, as shown in FIG. 22.

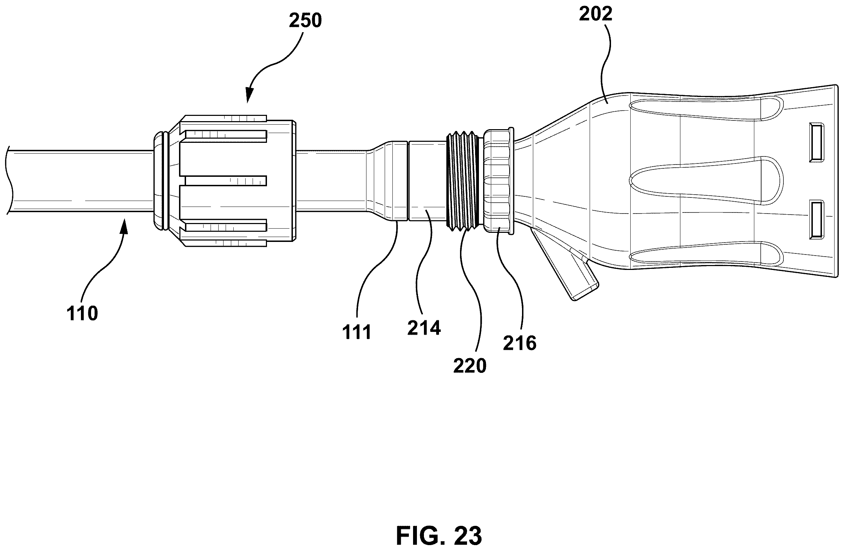

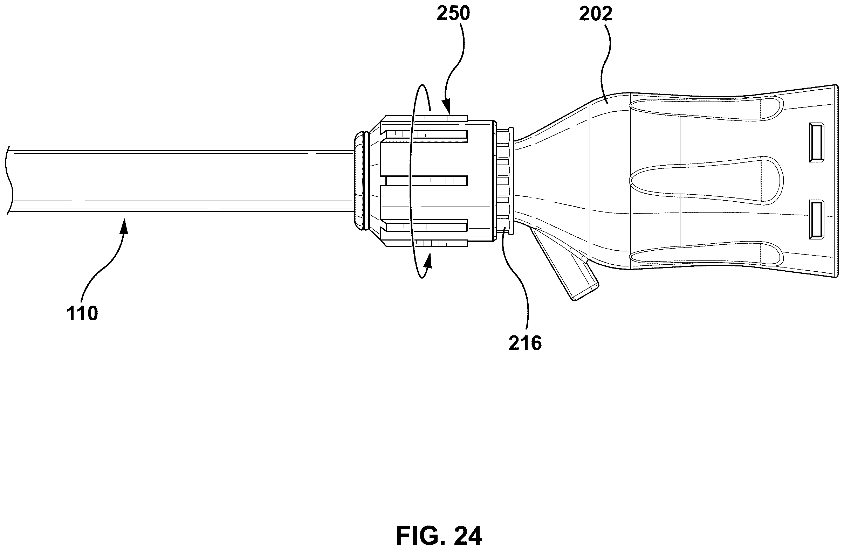

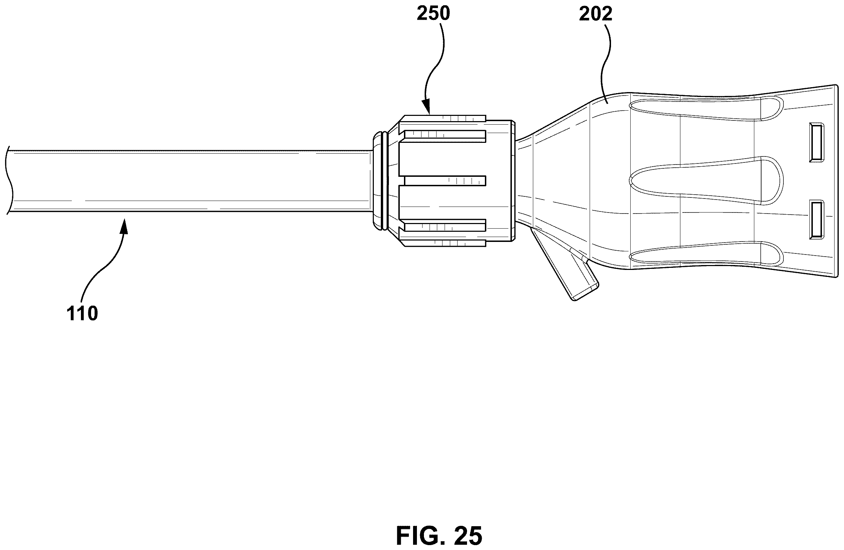

In another step of the method, as shown in FIG. 23, the cap 250 is placed over the sheath 110. In particular, the proximal end 254 of the cap 250 may be placed over the distal end of the sheath 110 and the cap 250 may be advanced proximally towards the hub body 202. In another step of the method, as shown in FIG. 24, the cap 250 is rotated relative to the hub body 202 such that the threads 270 of the cap 250 advance along and engage the threads 220 of the hub body 202. The cap 250 continues to be rotated and advanced proximally until the teeth 272 of the cap 250 lock onto two of the teeth 222 of the ratchet lock 216, as shown in FIG. 25. The cap 250 is thereby locked onto the hub body 202 and couples the sheath 110 to the hub 200, as shown in FIGS. 19 and 21.

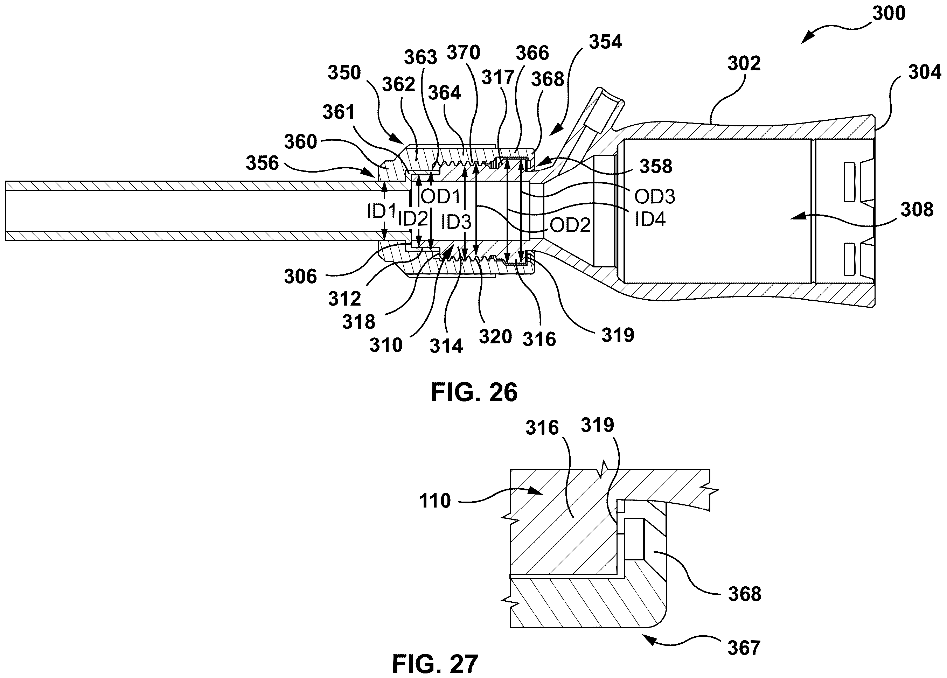

FIGS. 26-29 show devices and methods for securing a sheath of an introducer to an introducer hub 300 of the introducer. In embodiments, the sheath may be the sheath 110 as described above or may be other sheaths. In embodiments, the introducer hub 300 may be similar to the introducer hub 150 described above except for details described herein with respect to the introducer hub 300. However, this is not meant to be limiting, and the introducer hub 300 is not limited to the details described above with respect to the introducer hub 150.

FIG. 26 shows a longitudinal cross-sectional view of the introducer hub 300 with the sheath 110 coupled thereto according to embodiments herein. Those skilled in the art will recognize that all the details of the introducer hub 300 are not described and that other features may be included with the introducer hub 300 or that features may be excluded or modified. The introducer hub 300 includes a body 302 having a proximal end 304, a distal end 306, and a central lumen or passageway 308 extending from the proximal end 304 to the distal end 306. The introducer hub 300 may include seals, seal caps, and other features as described above or as known to those skilled in the art. The introducer hub 300 further includes a cap 350 for coupling the sheath 110 to the hub body 302.

In the embodiment shown, the hub body 302 of the introducer hub 300 includes a distal tube 310 extending proximally from the distal end 306 of the hub body 302. The distal tube 310 includes a first portion 312, a second portion 314 proximal of the first portion 312, and a third portion 316 proximal of the second portion 314. In particular, as shown in FIG. 26, the first portion 312 is a cylindrical tube with an inner surface thereof defining a portion of the central passageway 308. The wall of the first portion has a first wall thickness such that an outer surface thereof defines a first outer diameter OD1. The second portion 314 extends proximally from the first portion 312. The second portion 312 is a cylindrical tube defining a portion of the central passageway 308. The wall of the second portion 314 has a second wall thickness thicker than the first wall thickness such that an outer surface of the wall of the second portion defines a second outer diameter OD2 larger than the first outer diameter OD1. The change in wall thickness from the first portion 312 to the second portion 314 creates a shoulder 318 between the first portion 312 and the second portion 314. The second portion 314 further includes threads 320 along a portion thereof.

The third portion 316 is disposed proximal of the second portion 314. The third portion 314 is a cylindrical tube defining a portion of the central passageway 308. The wall of the third portion 314 has a second wall thickness thicker than the second wall thickness such that an outer surface of the wall of the third portion defines a third outer diameter OD3 larger than the second outer diameter OD2. The change in wall thickness from the second portion 314 to the third portion 316 creates a shoulder 317 between the second portion 314 and the third portion 316. At a proximal end of the third portion 316, the wall thickness is reduced, thereby creating a lip 319 for the cap 350 to lock onto, as described below.

The cap 350 of the introducer hub 300 is configured to couple the sheath 110 to the hub body 302, as described in more detail below. In the embodiment shown, the cap 350 includes a cap body 352 including a proximal end 354, a distal end 356, and a central lumen 358 extending from the proximal end 354 to the distal end 356. The cap 350 is a substantially cylindrical, stepped tube. Thus, in the embodiment shown, the cap body 352 includes a first longitudinal portion 360, a second longitudinal portion 362 proximal of the first longitudinal portion 360, a third longitudinal portion 364 proximal of the second longitudinal portion 362, and a fourth longitudinal portion 366 proximal of the third longitudinal portion 364. The first, second, third, and fourth longitudinal portions 360, 362, 364, 366 are defined by having different internal diameters. Thus, the first longitudinal portion 360 has an inner surface defining a portion of the central lumen 358 and having a first inner diameter ID1, the second longitudinal portion 362 has an inner surface defining a portion of the central lumen 358 and having a second inner diameter ID2, the third longitudinal portion 364 has an inner surface defining a portion of the central lumen 358 and having a third inner diameter ID3, and the fourth longitudinal portion 366 has an inner surface defining a portion of the central lumen 358 and having a fourth inner diameter ID4. In the embodiment shown, the first inner diameter ID1 is smaller than the second inner diameter ID2, which is smaller than the third inner diameter ID3, which is smaller than the fourth inner diameter ID4. Further, a first shoulder 361 is disposed at the inner surface between the first longitudinal portion 360 and the second longitudinal portion 362 and a second shoulder 363 is disposed at the inner surface between the second longitudinal portion 362 and the third longitudinal portion 364. Further, a lip 368 extends radially inwardly from the fourth longitudinal portion 366 at the proximal end 354 of the cap 350.

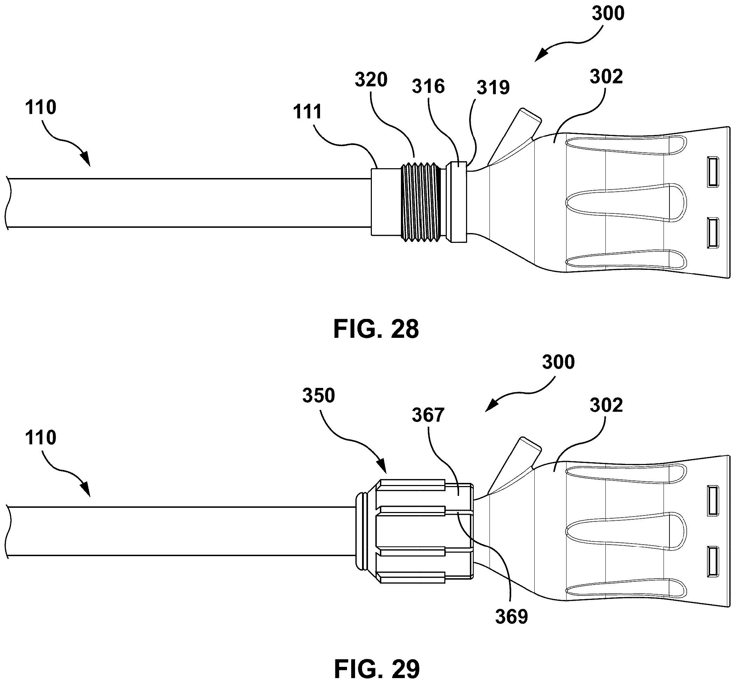

As shown in FIGS. 26 and 29, the fourth longitudinal portion 366 comprises a plurality of legs 367 extending longitudinally from third longitudinal portion 364. The legs 367 are separated circumferentially by gaps 369. Thus, the legs 367 are flexible such that the legs 367 may flex radially inwardly and radially outwardly. Further, the inner surface of the third longitudinal portion 364 includes threads 370 configured to engage the threads 320 of the distal tube 310 of the hub body 302.

As explained in more detail below, the cap 350 is configured to capture a proximal end of the sheath 110 between the cap body 352 and the hub body 302. Thus, in the embodiment shown, the first inner diameter ID1 of the cap body 352 is smaller than the first outer diameter OD1 of the hub body 302, the second inner diameter ID2 of the cap body 352 is larger than the first outer diameter OD1 of the hub body 302 but smaller than the second outer diameter OD2 of the hub body 302, the third inner diameter ID3 of the cap body 352 is larger than the second outer diameter of the hub body 302 but smaller than the third outer diameter OD3 of the hub body 302, and the fourth inner diameter ID4 of the cap body 352 is larger than the third outer diameter OD3 of the hub body 302, as shown in FIG. 26.

With reference to FIGS. 28-29, a method of coupling the sheath 110 to the introducer hub 300 will be described. In a step of the method, the flared proximal portion 111 of the sheath 110 is placed over the first portion 314 of the hub body 302, as shown in FIG. 28. In another step of the method, the cap 350 is placed over the sheath 110. In particular, the proximal end 354 of the cap 350 may be placed over the distal end of the sheath 110 and the cap 350 may be advanced proximally towards the hub body 302. In another step of the method, the cap 350 is rotated relative to the hub body 302 such that the threads 370 of the cap 350 advance along and engage the threads 320 of the hub body 302. The cap 350 continues to be rotated and advanced proximally until the lips 368 at the proximal ends 354 of the legs 367 of the cap 350 extends proximally past the proximal end of the third portion 316 of the distal tube 310 and engage lip 319 of the distal tube 310, as shown in FIGS. 26, 27, and 29. As the cap 350 is advanced proximally the legs 367 flex radially outwardly such that the lips 368 at the proximal end 354 of the legs 367 extend over the third portion 316 of the distal tube 310. As the cap 350 continues to be advanced proximally, the lips 368 clear the proximal end of the third portion 316 of the distal tube 310, and the legs 367 flex back radially inwardly to their original position such that the lips 368 of the legs 367 engage the lip 319 at the proximal end of the third portion 316 of the distal tube 310. This engagement of the lips 368 with the lip 319 locks the cap 350 in place, preventing the cap 350 from moving distally, and captures the flared proximal portion 111 of the sheath 110 between the cap 350 and the distal tube 310 of the hub body 302.

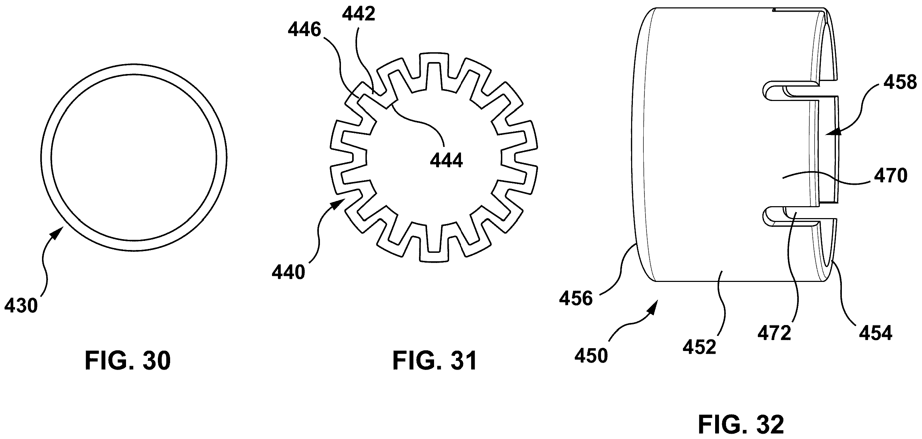

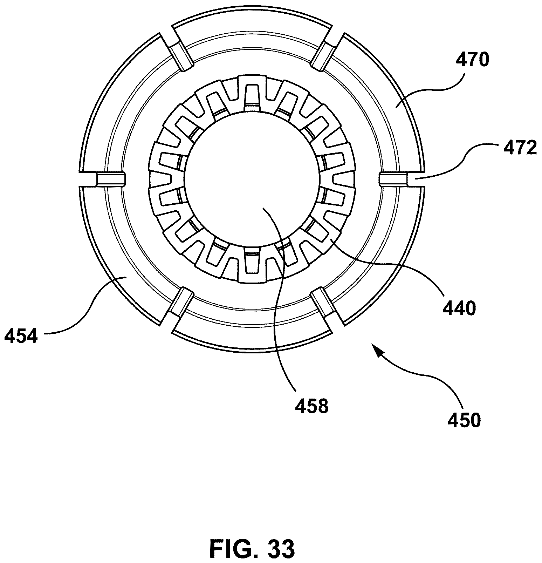

FIGS. 30-35 show devices and methods for securing a sheath of an introducer to an introducer hub 400 of the introducer. In embodiments, the sheath may be the sheath 110 as described above or may be other sheaths. In embodiments, the introducer hub 400 may be similar to the introducer hub 150 described above except for details described herein with respect to the introducer hub 400. However, this is not meant to be limiting, and the introducer hub 400 is not limited to the details described above with respect to the introducer hub 150.

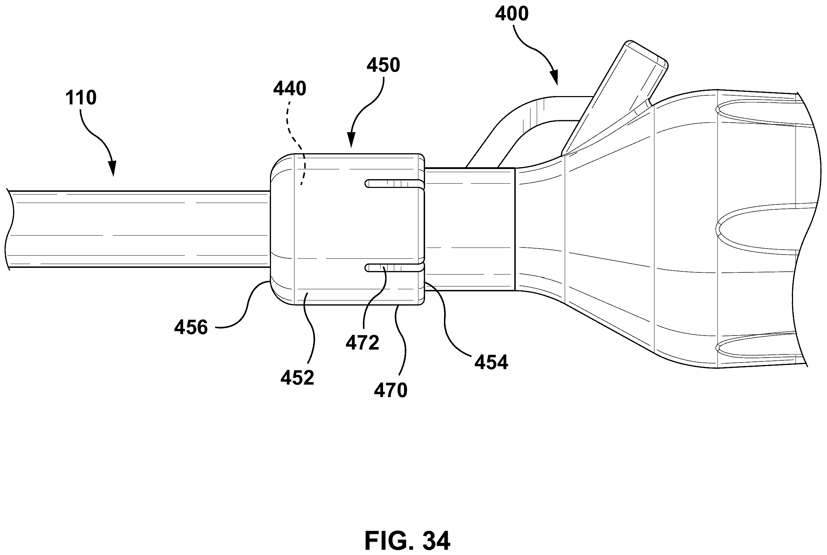

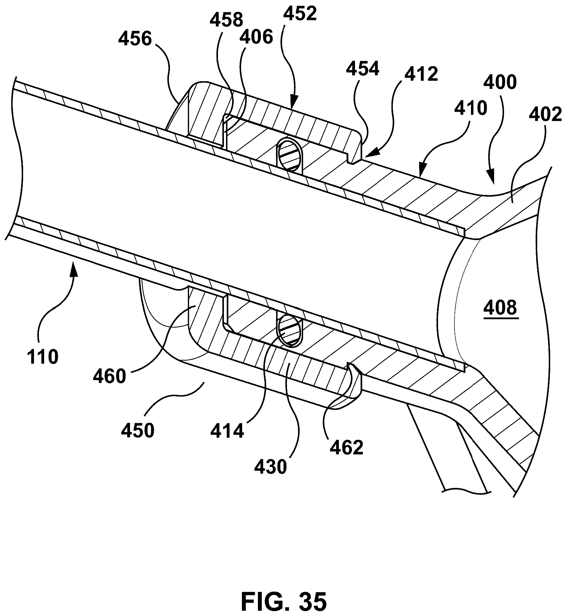

FIG. 34 shows the introducer hub 400 with an introducer sheath 110 coupled thereto via a connector 450. The connector 450 is shown in FIGS. 31 and 32 and includes a body or housing 452 having a proximal end 454, a distal end 456, and a central lumen 458 extending from the proximal end 454 to the distal end 456. The housing 452 further includes a plurality of legs 470 adjacent the proximal end 454 formed via gaps 472 between the legs 470. The gaps 472 enable the legs 470 to flex radially outwardly and inwardly, as discussed in more detail below. As best shown in FIG. 35, the proximal end 454 of the housing 452 includes a proximal lip 462 extending radially inward. Similarly, the distal end 456 of the housing 452 includes a distal lip 460 extending radially inwardly.

The connector 450 further includes a grab ring 440 as shown in FIG. 31. The grab ring 440 is a ring with a series of struts 442 extending radially outwardly and inwardly coupling first bends 444 disposed at radially inward ends of the struts 442 and second bends 446 disposed at radially outward ends of the struts 442. The grab ring 440 is disposed in the lumen 458 of the body 452 adjacent to the distal end 456 when the connector 450 couples the sheath 110 to the introducer hub 400.

In the embodiments, the introducer hub 400 includes a hub body 402 having a proximal end (not shown), a distal end 406, and a central lumen 408 extending therebetween. The hub body 402 includes a distal tube 410 extending proximally from the distal end 406 of the hub body. The distal tube 410 is a generally cylindrical tube defining a portion of the central lumen 408. The distal tube 410 further includes a first groove 412 extending circumferentially around an outer surface of the distal tube 410. The groove 412 extends radially inward from the outer surface of the distal tube 410. The distal tube 410 further includes a second groove 414 extending circumferentially around an inner surface of the distal tube 410. The second groove 414 extends radially outward from the inner surface of the distal tube 410 and is spaced distally of the first groove 412, as shown in FIG. 35.

The introducer of FIGS. 30-35 further includes an O-ring 430 as shown in FIG. 31.

With the parts of the introducer described, the connection between the sheath 110 and the introducer hub 400 will be described with reference to FIGS. 34 and 35. The O-ring 430 is disposed in the second groove 414 of the distal tube 410 and the proximal end of the sheath 110 is disposed within the distal tube 410 and within the O-ring 430. The O-ring 430 seals against the outer surface of the sheath 110 to prevent leakage between the sheath 110 and the inner surface of the distal tube 410.

The connector 450 with the grab ring 440 disposed in a distal portion of the central lumen 458 as advanced proximally over the sheath 110. As the housing 452 continues to be advanced proximally, the housing 452 extends over the distal tube 410 of the introducer hub 400. Because the proximal lip 462 extends radially inwardly, the legs 470 flex radially outwardly such that the housing 452 can be advanced over the distal tube 410. The housing 452 is advanced proximally until the proximal lip 462 reaches the first groove 412 of the distal tube 410, at which point the legs 470 flex inwardly back to their original position, thereby locking the housing 452 longitudinally with respect to the distal tube 410 via the proximal lip 462 extending into the first groove 412. The distal lip 460 of the housing 452 extends radially inwardly to contact the sheath 110. The distal lip 460 also presses the grab ring 440 against the distal end 406 of the distal tube 410.

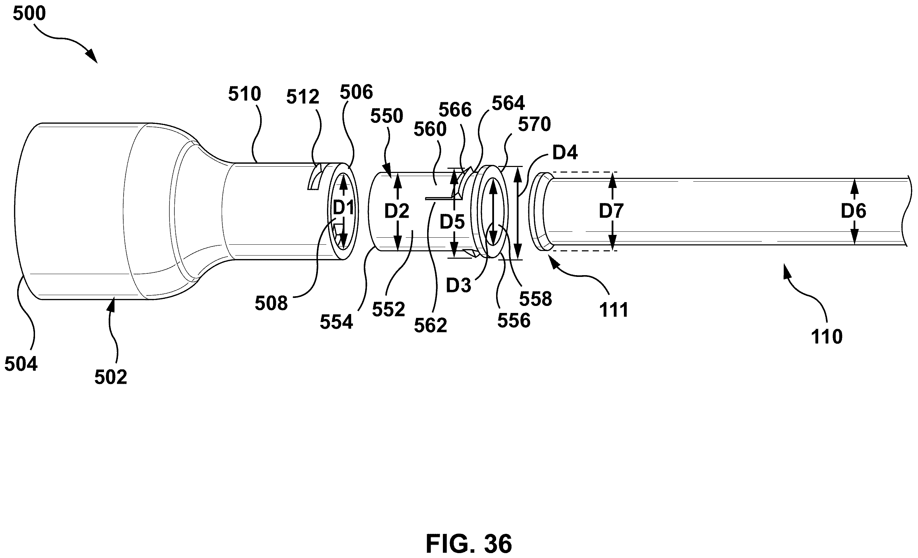

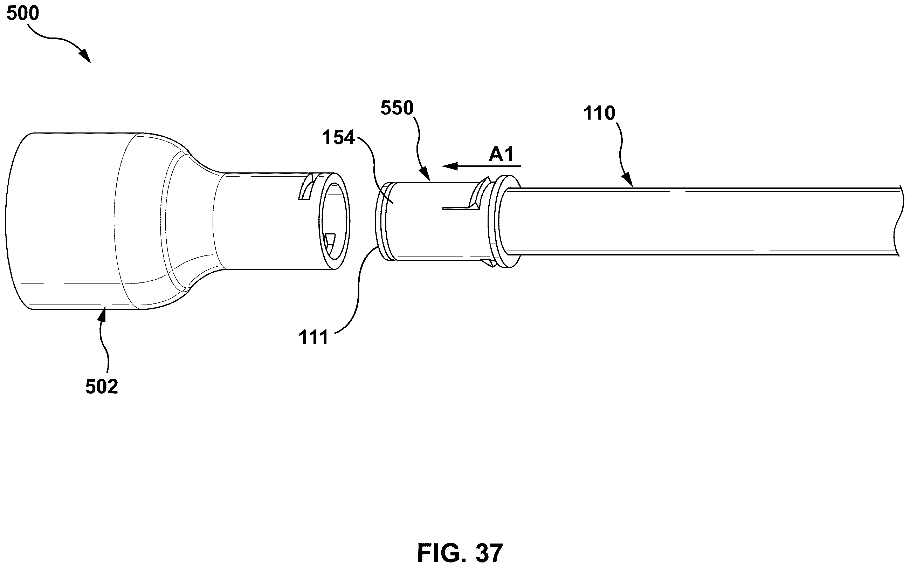

FIGS. 36-38 show devices and methods for securing a sheath of an introducer to an introducer hub 500 of the introducer. In embodiments, the sheath may be the sheath 110 as described above or may be other sheaths. In embodiments, the introducer hub 500 may be similar to the introducer hub 150 described above except for details described herein with respect to the introducer hub 500. However, this is not meant to be limiting, and the introducer hub 500 is not limited to the details described above with respect to the introducer hub 150.

FIG. 36 shows an exploded view of the introducer hub 500 and the sheath 110. Those skilled in the art will recognize that all the details of the introducer hub 500 are not described and that other features may be included with the introducer hub 500 or that features may be excluded or modified. Generally, the introducer hub 500 includes a hub body 502 and a locking clip 550. The hub body 502 defines a proximal end 504, a distal end 506, and a central lumen or passageway 508 extending from the proximal end 504 to the distal end 506. The introducer hub 500 may include seals, seal caps, and other features as described above or as known to those skilled in the art. The locking clip 550 is configured to couple the sheath 110 to the hub body 502, as described below. In embodiments, the locking clip 550 may be formed of GRILAMID or a similar material.

In the embodiment shown, the hub body 502 of the introducer hub 500 includes a distal tube 510 extending proximally from the distal end 506 of the hub body 502. The distal tube 510 may be a generally cylindrical tube with an inner surface defining a portion of the central passageway 508. An inner diameter D1 of the distal tube 510 or lumen diameter D1 of the central passageway 508 at the distal tube 510 is configured to receive the sheath 110 and the locking clip 550, as described below. The distal tube 510 further includes at least one slot 512 extending from an outer surface through the inner surface of the distal tube 510. The slot 512 is oriented circumferentially. In the embodiment shown, there are two slots 512 disposed opposite each other. However, this is not meant to be limiting, and in other embodiments there may be more or fewer slots 512.

The locking clip 550 is configured to couple the sheath 110 to the hub body 502, as described in more detail below. In the embodiment shown, the locking clip 550 is a generally cylindrical tube including a clip body 552 defining a proximal end 554, a distal end 556, and a central lumen 558 extending from the proximal end 554 to the distal end 556. The locking clip 550 further includes a lip 570 extending radially outward at the distal end 556 of the clip body 552. The locking clip 550 further includes at least one clip arm 560 proximal of the lip 570. In the embodiment shown, the locking clip 550 includes two clip arms 560 disposed opposite each other to match the slots 512 of the distal tube 510. A small portion of the second clip arm 560 can be seen in FIG. 36. The clip arms 560 are formed of a portion of the clip body 552 configured to flex radially inwardly and outwardly in reaction to forces acting thereupon. In particular, each clip arm 560 may be formed as a partially cutout of the clip body 552. In the embodiment shown, a first longitudinal cut 562 defines a first longitudinal edge of the clip arm 560, a distal circumferential cut 564 defines a distal edge of the clip arm 560, and a second longitudinal cut (not shown) opposite the first longitudinal cut 562 defines a second longitudinal edge of the clip arm 560. A proximal end of the clip arm 560 is coupled to the remainder of the clip body 552. Thus, the cantilever design of the clip arm 560 enables the clip arm 560 to flex radially inwardly and outwardly in response to forces acting thereupon. The clip arm 560 further includes an arm lip 566 extending radially outward at the distal end of the clip arm 560. The arm lip 566 extends circumferentially along the distal edge of the clip arm 560 and is configured to interact with one of the slots 512 of the hub body 502, as explained below.

An outer surface of the clip body 552 of the locking clip 550 defines an outer diameter D2. An inner surface of the clip body 552 defines an inner diameter D3 or lumen diameter D3 of the central lumen 558. An outer surface of the lip 570 defines an outer diameter D4. Further, outer surfaces of the arm lips 566 of clip arms 560 disposed opposite each other define an outer diameter D5 that may be equal to or slightly smaller than the outer diameter D4. Further the outer diameter D5 is larger than the inner diameter D1 of the central lumen 508 of the distal tube 510. Stated another way, an outer surface of one of the arm lips 566 of one of the clip arms 560 defines a radius from the central longitudinal axis of the central lumen 558, and that radius may be approximately half the outer diameter D5 or slightly larger than half the inner diameter D1 of the central lumen 508 of the distal tube 510.

The sheath 110 includes a sheath lip (flare proximal portion) 111 at a proximal end thereof. An outer surface of the sheath 110 defines an outer sheath diameter D6 and an outer surface of the sheath lip 111 defines an outer sheath lip diameter D7 that is larger than the outer sheath diameter D6.

With reference to FIGS. 27-28, a method of coupling the sheath 110 to the introducer hub 500 will be described. In a step of the method, the locking clip 550 is placed over the sheath 110. In particular, the proximal end 554 of the clip body 552 is aligned with a distal end of the sheath 110 and the locking clip 550 is slid over the sheath 110 such that the sheath 110 is located within the central lumen 558 of the locking clip 550. Thus, the outer sheath diameter D6 is smaller than the lumen diameter D3 of the central lumen 558 of the locking clip 550. As indicated by arrow A1, the locking clip 550 and/or the sheath 110 are translated relative to each other such that the locking clip 550 translates proximally relative to the sheath 110, as shown in FIG. 27. The sheath lip 111 at the proximal end of the sheath 110 prevents the locking clip 550 from translating proximal of the sheath lip 111. In other words, in the embodiment shown, the outer sheath lip diameter D7 is larger than the lumen diameter D3 of the central lumen 558 of the locking clip 550 such that the proximal end 554 of the locking clip abuts against the sheath lip 111.

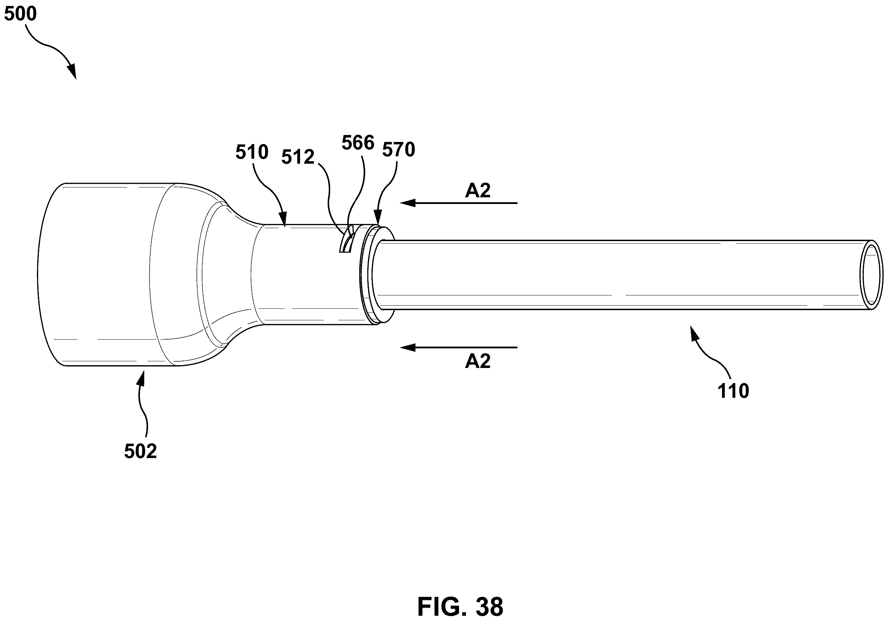

In another step of the method, the sheath 110 and locking clip 550 are translated proximally relative to the hub body 502 as indicated by the arrows A2 in FIG. 38 such that the proximal ends of the sheath 110 and the locking clip 550 are inserted into the central lumen 508 of the distal tube 510. Those skilled in the art will recognize that the translation is directed to relative movement, so the sheath/locking clip 110/550 can be translated proximally, the hub body 501 can be translated distally, or some combination of proximal and distal translation may be utilized. With the proximal ends of the sheath 110 and the locking clip 550 being inserted into the central lumen 508 of the hub body 502, the outer sheath lip diameter D7 and the outer clip diameter D2 are both smaller than the lumen diameter D1 of the central lumen 508 at the distal tube 510. However, the outer diameter D5 at the arm lips 566 is larger than the lumen diameter D1 of the central lumen 508 of the distal tube 510. Thus, the clip arms 560 flex radially inwardly such that the sheath 110/locking clip 550 combination may be inserted into the central lumen 508 of the distal tube 510. When the sheath 110/locking clip 550 combination is inserted into the central lumen 508 of the distal tube 510 a defined distance, the arms lips 566 of the clip arms 560 align with the slots 512 of the distal tube 510, thereby enabling the clip arms 560 to flex back to the original radial position as the arm lips 566 extend radially into the slots 512. The relative longitudinal locations of the clip arms 560, arms lips 566, and slots 512 are such that when the arm lips 566 extend into the slots 512, the lip 570 at the distal end 556 of the locking clip 550 abuts against the distal end 506 of the distal tube 510. Thus, the outer diameter D4 of the lip 570 of the locking clip 550 is larger than the lumen diameter D1 of the central lumen 508 of the distal tube 510. With the method as described completed, the sheath 110 is coupled to the distal tube 510 of the hub body 502 and locked into place by the locking clip 550.

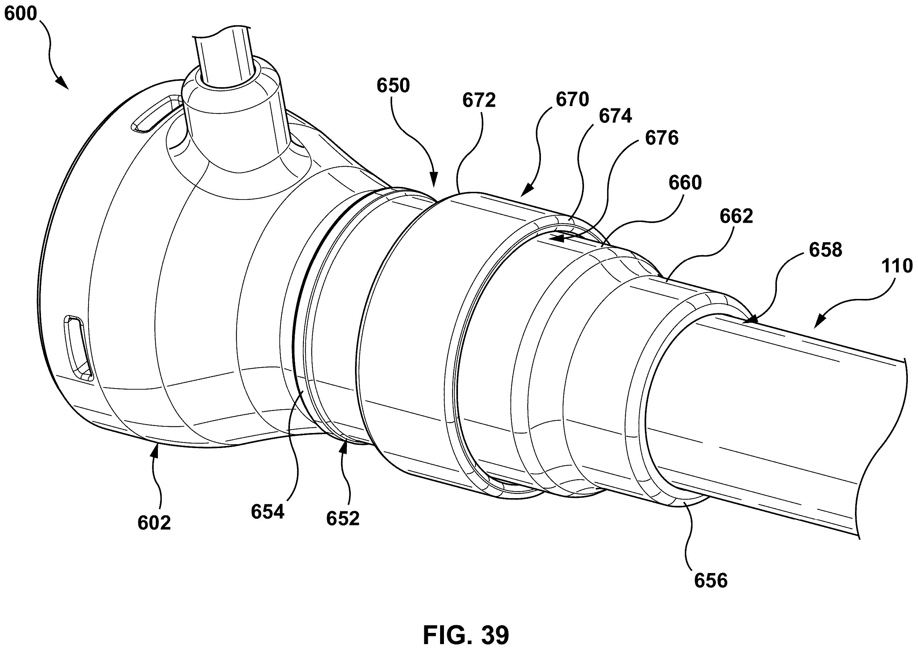

FIGS. 39-41 devices and methods for securing a sheath of an introducer to an introducer hub 600 of the introducer. In embodiments, the sheath may be the sheath 110 as described above or may be other sheaths. In embodiments, the introducer hub 600 may be similar to the introducer hub 150 described above except for details described herein with respect to the introducer hub 600. However, this is not meant to be limiting, and the introducer hub 600 is not limited to the details described above with respect to the introducer hub 150.

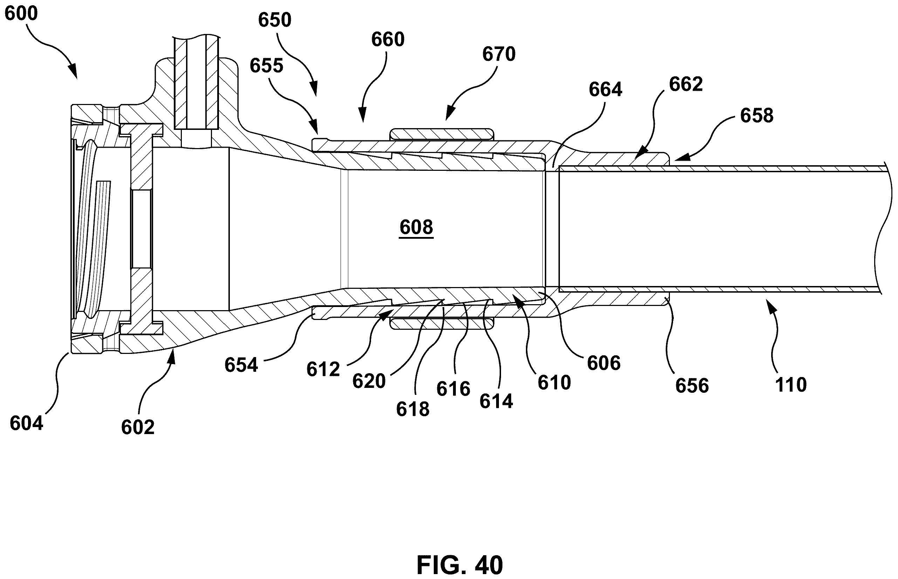

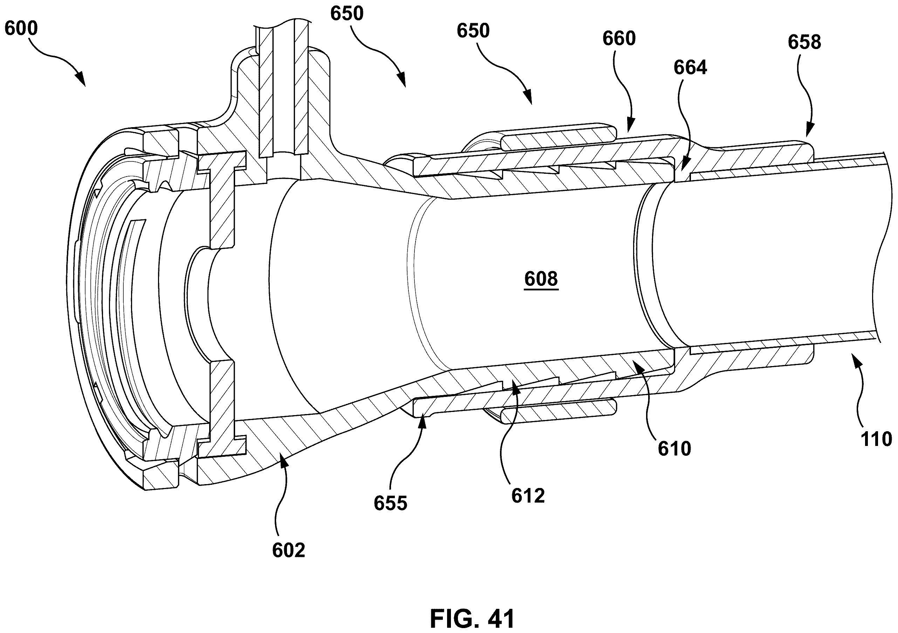

FIG. 39 shows a perspective view of the introducer hub 600 with the sheath 110 coupled thereto by a connector 650. FIG. 40 shows a longitudinal cross-sectional view of the sheath 110 coupled to the introducer hub 600 by the connector. FIG. 41 shows a perspective view of a longitudinal cross-section of the sheath 110 coupled to the introducer hub 600 via the connector 650. Those skilled in the art will recognize that all the details of the introducer hub 600 are not described and that other features may be included with the introducer hub 600 or that features may be excluded or modified.

Generally, the introducer hub 600 includes a hub body 602 defining a proximal end 604, a distal end 606, and a central lumen or passageway 608 extending from the proximal end 604 to the distal end 606. The introducer hub 600 may include seals, seal caps, and other features as described above or as known to those skilled in the art. The connector 650 is configured to couple the sheath 110 to the hub body 602, as described below. Further, a band or strap 670 surrounds a portion of the connector 650 to couple the connector to the introducer hub 600.

In the embodiment shown, the hub body 602 of the introducer hub 600 includes a distal tube 610 extending proximally from the distal end 606 of the hub body 602. The distal tube 610 may be a generally cylindrical tube with an inner surface defining a portion of the central passageway 608. In the embodiment of FIGS. 39-41, the distal tube 610 includes barbs 612 extending radially outwardly along the distal tube 610. In the embodiment shown, the distal tube includes three barbs 612 extending radially outwardly and circumferentially around the circumference of the distal tube 610. In particular, each barb 612 includes a first, distal end 614. A ramp 616 extends radially outwardly and proximally from the first, distal end 614 to a second, proximal end 618 of the barb 612. At the second, proximal end 614, the barb 612 extends radially inwardly, forming a shoulder 620 at the proximal end of the barb 612.

The connector 650 is configured to couple the sheath 110 to the hub body 602, as described in more detail below. In the embodiment shown, the connector 650 is a generally cylindrical tube including a body 652 defining a proximal end 654, a distal end 656, and a central lumen 658 extending from the proximal end 654 to the distal end 656. The connector 650 includes a proximal portion 660, a distal portion 662, and a lip 664 between the proximal portion 660 and the distal portion 662. The central lumen 658 is continuous through the proximal portion 660 and the distal portion 662.

As best shown in FIGS. 40 and 41, the distal portion 662 of the connector 650 is configured to receive a proximal portion of the sheath 110. In particular, the distal portion 662 of the connector 650 is overmolded over the proximal portion of the sheath 110. Thus, in embodiments herein, the material of the connector 650, or at least the distal portion 662 of the connector 650, is the same as or compatible with the material of an outer layer of the proximal portion of the sheath 110 such that overmolding will attach the proximal portion of the sheath 110 to the distal portion 662 of the connector 650. In a non-limiting example, the material of the connector 650 may be a polyether-based thermoplastic polyurethane such as, but not limited to, Texin^{®} 85A. However, other suitable materials known to those skilled in the art may also be used.

As also shown in FIGS. 40 and 41, the proximal portion 660 of the connector 650 is disposed over the distal tube 610 of the hub body 602. In particular, the proximal portion 660 of the connector 650 extends proximally over the distal tube 610 from the distal end 606 of the distal tube 610 and covers the barbs 612 of the distal tube 610. The proximal end 654 of the connector 650 may include a ridge 655 for increased strength of the connector at the proximal end 654.

The band 670 is configured to be disposed around the proximal portion 660 of the connector 650 to couple the connector 650 to the distal tube 610 of the hub body 602. In an embodiment, the band 670 is a generally cylindrical tube including a proximal end 672, a distal end 674, and a lumen 676 extending from the proximal end 672 to the distal end 674. The lumen 676 is sized such that the band 670 applies a radially inward force on the proximal portion 660 of the connector 650 to deform the material of the proximal portion 660 onto the barbs 612 of the distal tube 610. In a non-limiting example, the band 670 may be formed of stainless steel and the proximal portion 660 of the connector 650 may be formed of a polyether-based thermoplastic polyurethane such as, but not limited to, Texin^{®} 85A. However, those skilled in the art will recognize that other materials may be used.

To attach the sheath 110 to the introducer hub 600, the distal portion 662 of the connector 650 is overmolded over the proximal portion of the sheath 110. The proximal portion 660 of the connector 650 is translated over the distal tube 610 of the hub body 602. The orientation of the barbs 612 extending proximally assists in the proximal portion 660 extending over the barbs 612. The band 670 is then translated proximally over the proximal portion 660 of the connector 650, applying an inward force to the proximal portion 660. The orientation of the barbs 612 as described above assists in the band 670 advancing proximally. Further, once connected, the orientation of the barbs 612 resists distal movement of the proximal portion 660 of the connector 650 such that the connection between the sheath 110 and the hub 600 is secure.

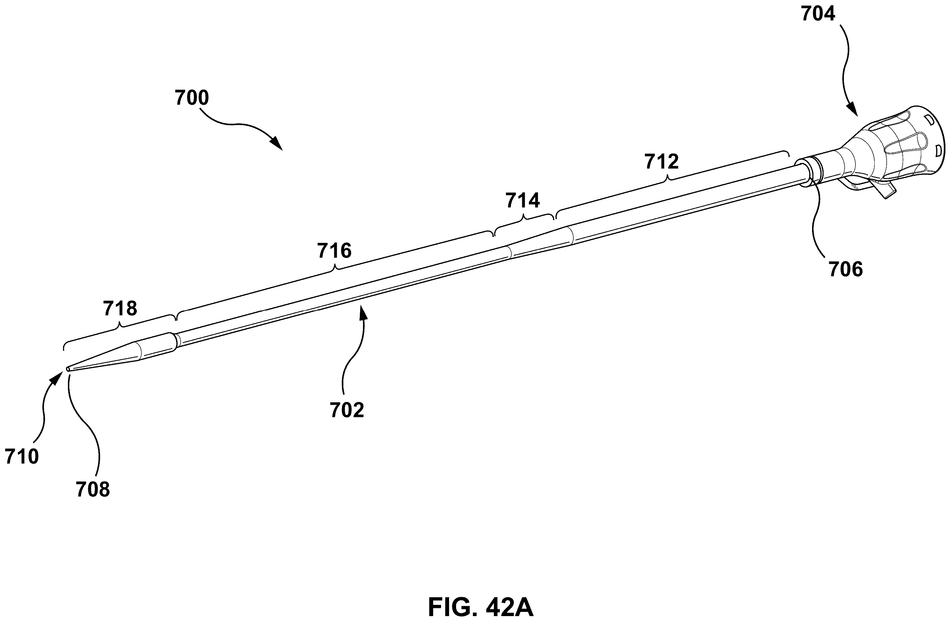

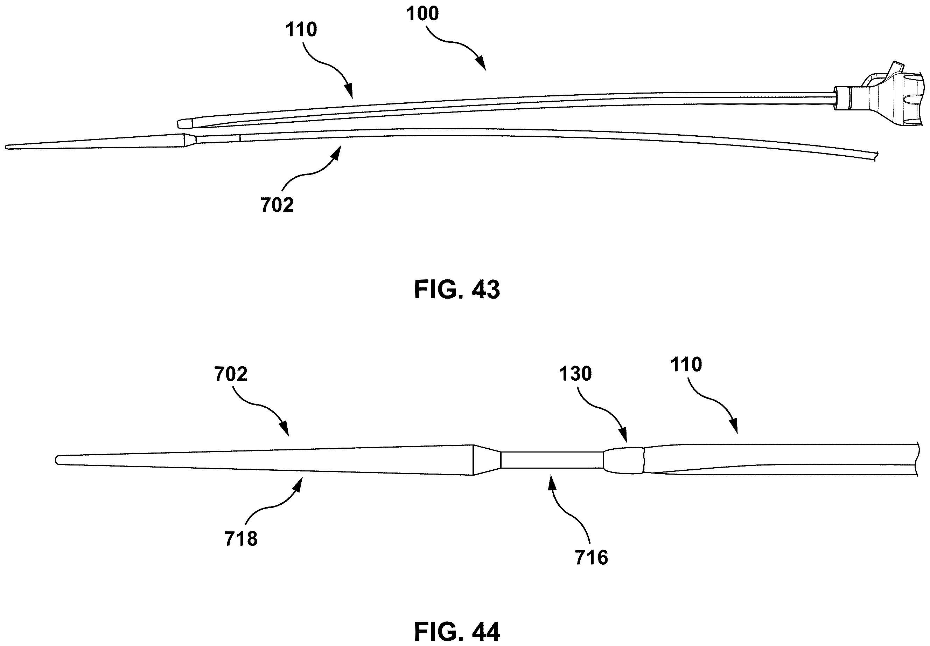

FIGS. 42A-50 show a dilator 700 according to embodiments hereof. The dilator 700 is configured to be used with an expandable introducer, such as, but not limited to the expandable introducers described herein, and in particular, to the expandable sheath described with respect to FIGS. 1-16 hereof. However, this is not meant to be limiting and the dilator 700 may be used with other expandable introducers and other expandable sheaths thereof. Those skilled in the art will recognize that features may be added and/or removed from the dilator 700.

The dilator 700 generally includes a dilator shaft 702 coupled to a dilator hub 704. Details of the dilator hub 704 and the connector of the dilator shaft 702 to the dilator hub 704 are not described herein. Accordingly, dilator hubs known to those skilled in the art may be utilized with the dilator shaft 702 described herein.

The dilator shaft 702 includes a proximal end 706 for coupling to the dilator hub 704, a distal end 708, and a central lumen 710 extending from the proximal end 706 to the distal end 708. The dilator shaft 702 further includes a proximal portion 712, a proximal transition portion 714, a middle portion 716, and a distal portion 718. The lengths and diameters of the portions described herein are configured to interact with an expandable sheath such as the expandable sheath 110 described above. Accordingly, lengths and/or diameters described herein may be described as relative to portions of the expandable sheath 110 such that different sized dilators may be used with different sized expandable sheaths.

The proximal portion 712 of the dilator shaft 702 is configured to have a maximum outer diameter while still fitting within the introducer hub such as the introducer hub 150 described above. In an embodiment, the outer diameter of the proximal portion 712 of the dilator shaft 702 may be about 6 mm for a nominal 18 French dilator. Further, in an embodiment, the proximal portion 712 may have a length of about 200 mm.

The middle portion 716 of the dilator shaft 702 has a reduced outer diameter as compared to the proximal portion 712. Thus, in an embodiment, the middle portion 716 has an outer diameter of about 4 mm for an 18 French dilator. However, as noted above, the diameters may vary depending on the application of the dilator and the introducer used. Thus, in an embodiment, the outer diameter of the middle portion 716 of the dilator shaft 702 is smaller than an inner diameter D3 of the introducer sheath 110 when the introducer sheath 110 is in the radially unexpanded, folded state described above. Thus, in the embodiment described, the middle portion 716 of the dilator shaft 702 will not radially expand the expandable sheath 110 when the middle portion 716 is disposed within the expandable sheath 110. In an embodiment, the middle portion 716 of the dilator shaft 702 may have a length of about 285 mm.

The proximal transition portion 714 is disposed between the proximal portion 712 and the middle portion 716. The outer diameter of proximal transition portion 714 tapers in a distal direction from the outer diameter of the proximal portion 712 at the proximal end of proximal transition portion 714 to the outer diameter of the middle portion 716 at the distal end of the proximal transition portion 714. Thus, in an example embodiment, the outer diameter of the proximal transition portion 714 tapers from about 6 mm at its proximal end to about 4 mm at its distal end over a length of about 30 mm from the its proximal end to its distal end. However, as explained above, this is not meant to be limiting.

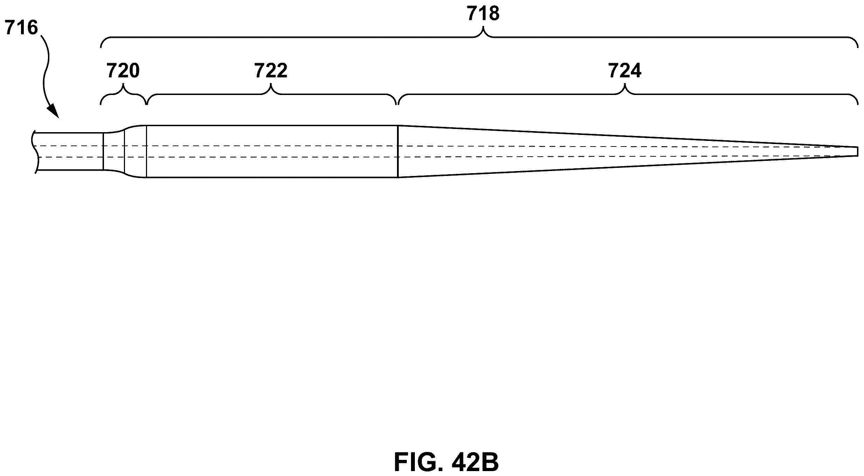

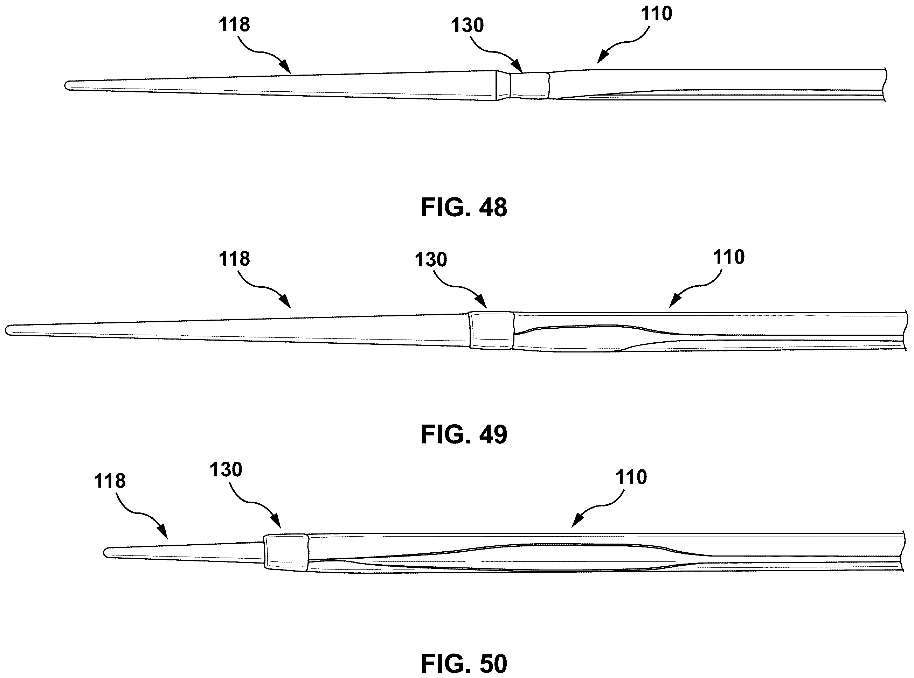

The distal portion 718 of the dilator shaft 702 extends distally from the middle portion 716. As shown in FIG. 42B, the distal portion 718 may comprise a distal transition portion 720, a distal cylindrical portion 722, and a distal tapered tip 724. The distal transition portion 720 flares distally from the outer diameter of the middle portion 716 to the outer diameter of the distal cylindrical portion 722. The distal cylindrical portion 722 extends distally from the distal transition portion 720. The distal cylindrical portion 722 is sized such that when the distal cylindrical portion 722 is retracted proximally through the expandable sheath 110, the distal cylindrical portion 722 pre-dilates or conditions the expandable sheath 110 for radially expansion, as shown in FIGS. 48-50. Thus, in an example, the outer diameter of the distal cylindrical portion 722 approximately equals the outer diameter of the expandable sheath 110 in the radially unexpanded, folded state. In an example, the outer diameter of the distal cylindrical portion 722 is about 5.8 mm. In an example, the distal cylindrical portion 722 has a length of about 30 mm.

The distal tapered tip 724 extends distally from the distal cylindrical portion 722. The distal tapered tip 724 tapers in a distal direction from a first outer diameter at its proximal end to a second outer diameter smaller than the first outer diameter at its distal end. The distal tapered tip 724 is configured for atraumatic insertion of the dilator into a vessel. In an embodiment, the outer diameter of the distal tapered tip 724 at its proximal end is about 5.8 mm and the outer diameter of the distal tapered tip at its distal end is about 1.28 mm. Further, in an embodiment, the distal tapered tip 724 has a length of about 55 mm.

The central lumen 710 is defined by an inner diameter of the shaft 702. The central lumen 710 may have a relatively constant diameter through the proximal portion 712, the proximal transition portion 714, the middle portion 716, and a portion of the distal portion 718. In an embodiment, the diameter of the central lumen is about 1.5 mm. In an embodiment, a distal portion of the central lumen 710 tapers in a distal direction. In an example, a distal 50 mm of the central lumen 710 tapers from a diameter of about 1.5 mm to a diameter of about 0.94 mm. Then, a final 12 mm of the central lumen remains a constant diameter of about 0.94 mm. Those skilled in the art will recognize that the sizes noted are merely examples of a particular embodiment of the dilator shaft 702, and are not meant to be limiting.

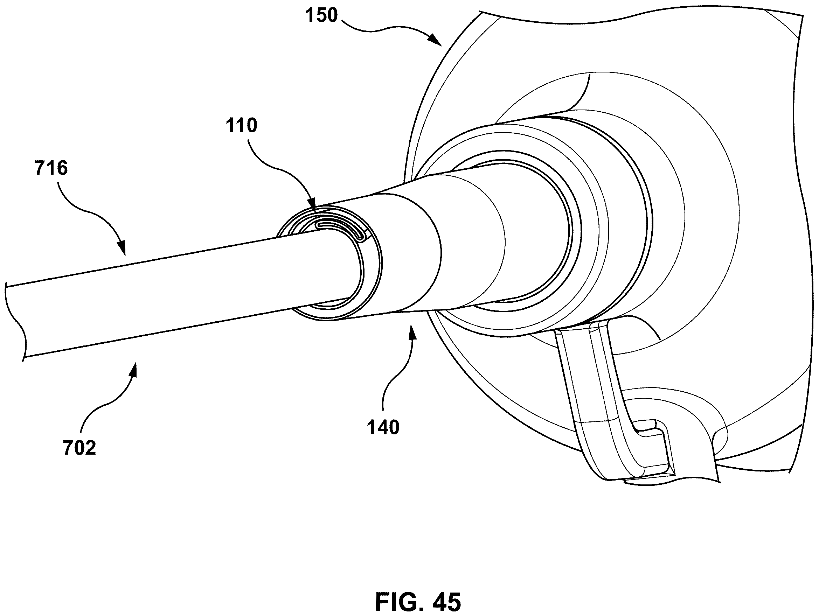

Having described the dilator shaft 702 with respect to the expandable sheath 110, in an embodiment, the proximal portion 712 and the proximal transition portion 714 of the dilator shaft 702 may initially not be coupled to the middle portion 716. Thus, in such an embodiment, the middle portion 716 may be inserted into a distal end of the expandable sheath 110 and advanced proximally therethrough. The proximal transition portion 714, the proximal portion 712, and the dilator hub 704 may then be coupled to the middle portion 716. In such a method, the introducer sheath 110 may remain in the radially unexpanded, folded state during insertion into a patient's vessel, as shown in FIG. 45, and will be radially expanded only upon proximal retraction of the dilator shaft 702 through the expandable sheath 110.

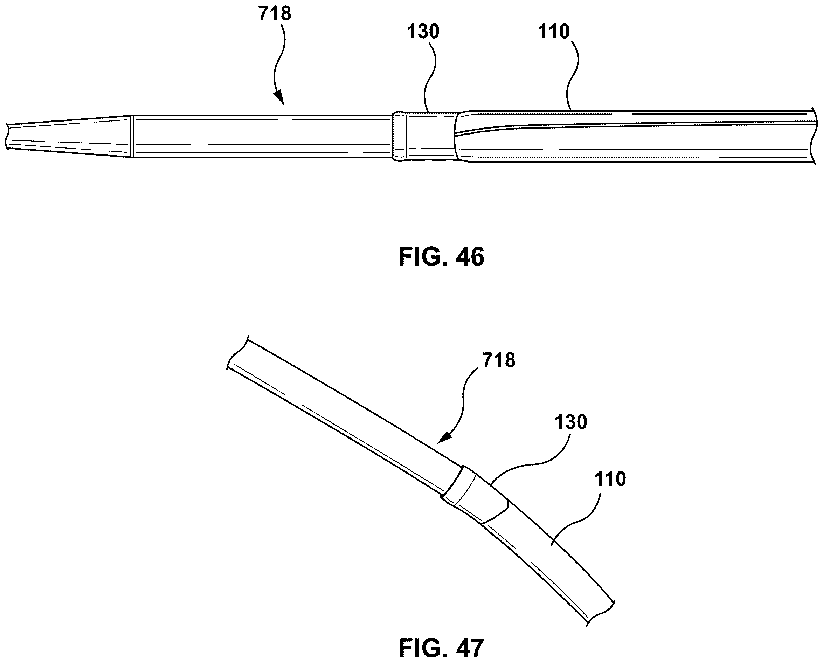

FIGS. 46 and 47 show the dilator shaft 702 disposed through the introducer sheath 110 with the distal "bump" (proximal portion of the distal cylindrical portion 722) disposed within the tip 130 of the expandable sheath 110. By placing the distal bump within the tip 130, the tip 130 stretches. The stretching of the tip 130 provides a smaller profile of the tip 130, thereby reducing the likelihood of the tip 130 catching within the vessel. In other words, the distal end of the tip 130 is stretched such that the distal end presents a thinner edge than when the distal end of the tip 130 is not stretched.

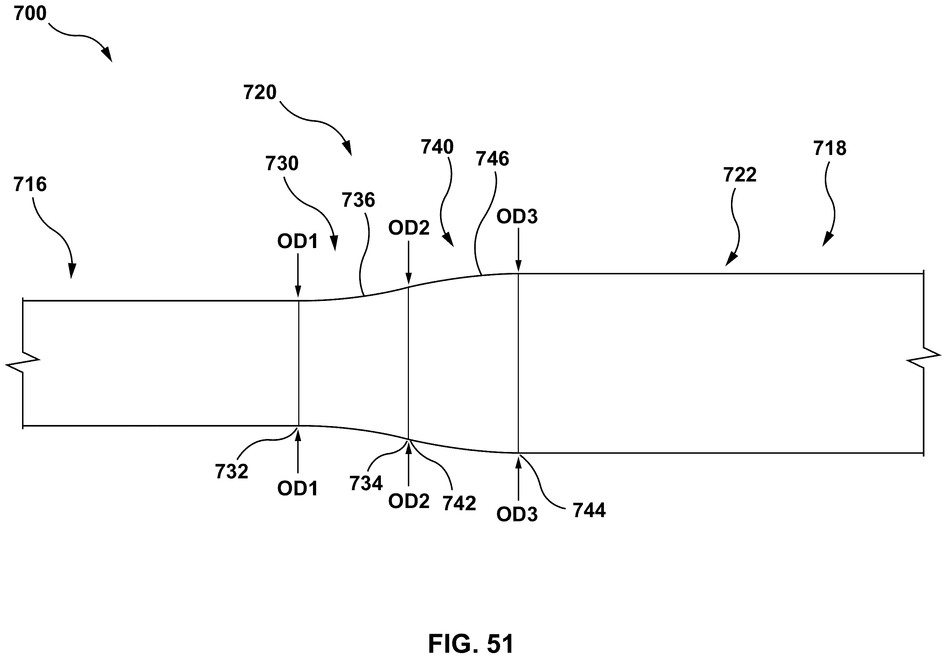

FIGS. 51-58 show distal transition portions of a dilator, such as the dilator 700 described previously. In the embodiments herein, the distal transition portions are configured to smooth or flatten the dilator to sheath transition, thereby providing a reduced likelihood of a tip of the sheath catching on tissue within the vessel and improving atraumaticity of the dilator to introducer sheath transition.

FIGS. 51-52 show the distal transition portion 720 of the dilator 700, according to an embodiment of the present disclosure. The distal transition portion 720 includes a proximal or a first section 730 and a distal or a second section 740. The first section 730 of the distal transition portion 720 includes a proximal end 732 and a distal end 734, as shown in FIG. 51. The proximal end 732 is disposed adjacent to a distal end of the middle portion 716 of the dilator 700 and extends distally to the distal end 734. In embodiments herein, a length of the first section 730 of the distal transition portion 720 may be in the range of 2mm to 15mm. The first section 730 of the distal transition portion 720 has a first outer diameter OD1 at the proximal end 732 and a second outer diameter OD2 at the distal end 734, wherein the first outer diameter OD1 is smaller than the second outer diameter OD2. The first outer diameter OD1 may be in the range of about 2mm to about 10mm. The second outer diameter OD2 may be in the range of about 2.5mm to about 11mm. An outer surface 736 of the first section 730 flares outwardly and distally in a concave profile from the first outer diameter OD1 at the proximal end 732 to the second outer diameter OD2 at the distal end 734.

The second section 740 of the distal transition portion 720 includes a proximal end 742 and a distal end 744. The proximal end 742 is disposed adjacent to the distal end 734 of the first section 730. The second section 740 extends distally from the proximal end 742 to the distal end 744. The distal end 744 is disposed adjacent to a proximal end of the distal cylindrical portion 722 of the distal portion 718 of the dilator 700. The length of the second section 740 of the distal transition portion 720 may be in the range of 2mm to 10mm. The second section 740 has the second outer diameter OD2 at the proximal end 742 and a third outer diameter OD3 at the distal end 744. In embodiments herein, the second outer diameter OD2 is smaller than the third outer diameter OD3. The third outer diameter OD3 may be in the range of about 3mm to about 12mm. An outer surface 746 of the second section 740 flares outwardly and distally in a convex profile from the second outer diameter OD2 at the proximal end 742 to the third outer diameter OD3 at the distal end 744.

Thus, as shown in FIG. 51, the distal transition portion 720 flares radially outward and is curved. As described, the first section 730 curves such that the outer surface 736 is concave when viewed from outside of the dilator 700 and the second section 740 curves such that the outer surface 746 is convex when viewed from outside of the dilator 700. The embodiment of FIGS. 51-52 may also be referred to as rounded.

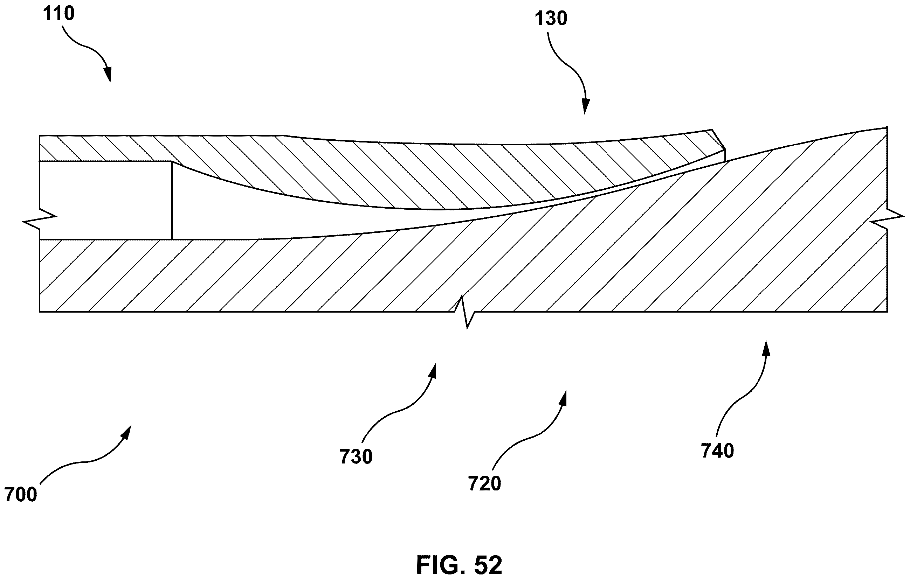

FIG. 52 shows a distal portion of the introducer sheath 110 disposed on an outer surface of the dilator 700, with the tip 130 of the introducer sheath 110 disposed on the first section 730 and a portion of the second section 740 of the distal transition portion 720 of the dilator 700. By placing the tip 130 on the first section 730 and a portion of the second section 740 of the distal transition portion 720 of the dilator 700, the transition between the tip 130 and the second section 740 of the distal transition portion 720 of the dilator 700 is generally smooth. Thus, the rounded shape of the distal transition portion 720 of the dilator 700 provides a reduced likelihood of the tip 130 of the introducer sheath 110 catching on tissue within the vessel.

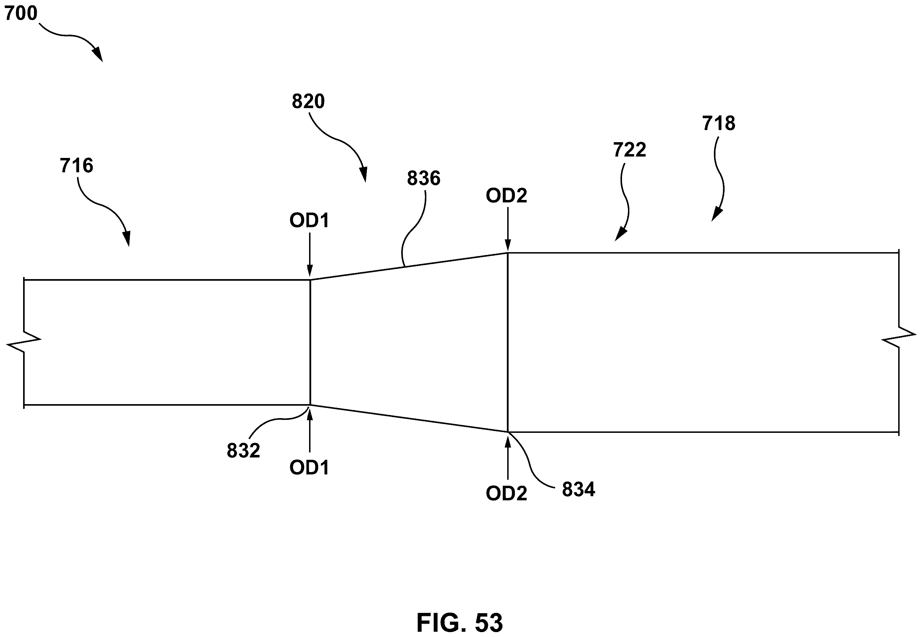

FIGS. 53-54 show a distal transition portion 820 according to another embodiment hereof. The distal transition portion 820 may be used with embodiments of the dilator 700 described above. In the embodiment of FIGS. 53-54, the distal transition portion 820 includes a proximal end 832 and a distal end 834. The proximal end 832 is disposed adjacent to the distal end of the middle portion 716 of the dilator 700 and extends distally to the distal end 834, which is disposed adjacent to a proximal end of the distal cylindrical portion 722 of the distal portion 718 of the dilator 700. In embodiments herein, a length of the distal transition portion 820 may be in the range of 2mm to 20mm. The distal transition portion 820 has a first outer diameter OD1 at the proximal end 832 and a second outer diameter OD2 at the distal end 834, wherein the first outer diameter OD1 is smaller than the second outer diameter OD2. In embodiments herein, the outer surface 836 of the first section 830 is flared from the proximal end 832 to the distal end 834. The first outer diameter OD1 may be in the range of about 2mm to about 10mm. The second outer diameter OD2 may be in the range of 3mm to 12mm. In the embodiment of FIGS. 53-54, the outer surface 836 of the distal transition portion 820 flares linearly from the proximal end 832 to the distal end 834. The distal transition portion 820 may also be referred to as a straight or linear transition portion.

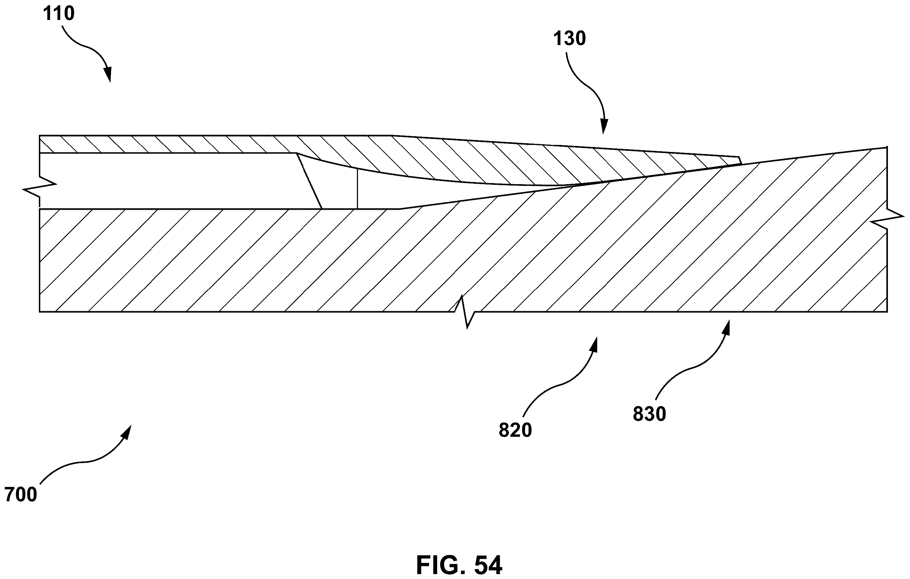

FIG. 54 shows a distal portion of the introducer sheath 110 disposed on an outer surface of the dilator 700. The tip 130 of the introducer sheath 110 is disposed on the distal transition portion 820 of the dilator 700. Placing the tip 130 on the distal transition portion 820 proximal of the distal cylindrical portion 722 provides a generally smooth transition between the tip 130 and the distal cylindrical portion 722 of the dilator 700 and thereby reduces the likelihood of the tip 130 catching tissue within the vessel.

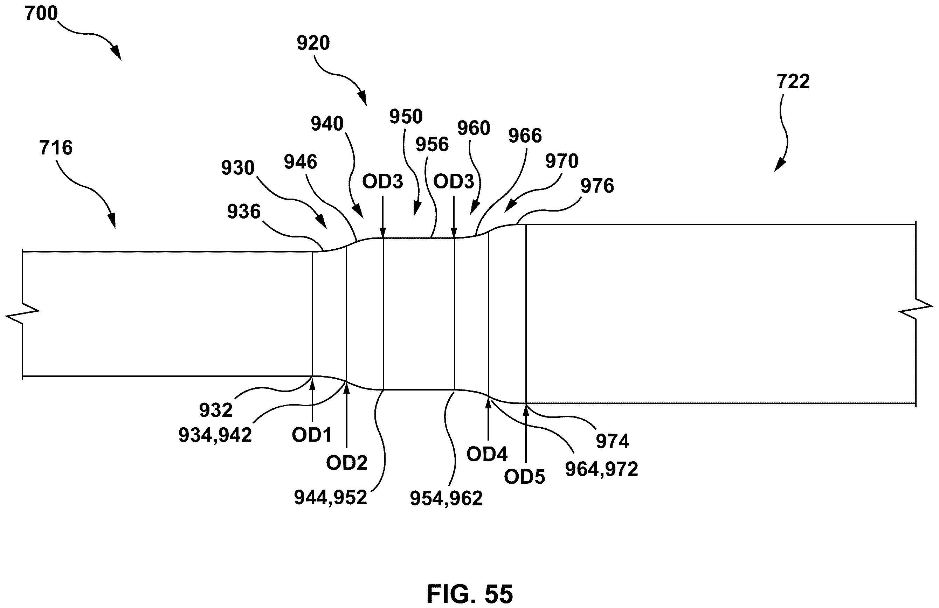

FIGS. 55-56 show a distal transition portion 920 according to another embodiment hereof. The distal transition portion 920 may be used with embodiments of the dilator 700 described above. In the embodiment of FIGS. 55-56, the distal transition portion 920 includes a first section 930, a second section 940, a third section 950, a fourth section 960, and a fifth section 970.

The first section 930 of the distal transition portion 920 includes a proximal end 932 and a distal end 934. The proximal end 932 is disposed adjacent to the distal end of the middle portion 716 of the dilator 700 and extends distally to the distal end 934. The length of the first section 930 of the distal transition portion 920 may be in the range of about 1mm to about 5mm. The first section 930 of the distal transition portion 920 has a first outer diameter OD1 at the proximal end 932 and a second outer diameter OD2 at the distal end 934. In embodiments herein, the first outer diameter OD1 is smaller than the second outer diameter OD2. The first outer diameter OD1 may be in the range of about 2mm to about 10mm. The second outer diameter OD2 may be in the range of about 2.25mm to about 10.5mm. In embodiments herein, an outer surface 936 of the first section 930 flares outwardly and distally in a concave profile from the first outer diameter OD1 at the proximal end 932 to the second outer diameter OD2 at the distal end 934.

The second section 940 of the distal transition portion 920 includes a proximal end 942 and a distal end 944. The proximal end 942 is disposed adjacent to the distal end 934 of the first section 930. The second section 940 extends distally from the proximal end 942 to the distal end 944. The length of the second section 940 may be in the range of about 1mm to about 5mm. The second section 940 has the second outer diameter OD2 at the proximal end 942 and a third outer diameter OD3 at the distal end 944. In embodiments herein, the second outer diameter OD2 is smaller than the third outer diameter OD3. The third outer diameter OD3 may be in the range of about 2.5mm to about 11mm. In embodiments herein, an outer surface 946 of the second section 940 flares outwardly and distally in a convex profile from the second outer diameter OD2 at the proximal end 942 to the third outer diameter OD3 at the distal end 944.

The third section 950 of the distal transition portion 920 includes a proximal end 952, disposed adjacent to the distal end 944 of the second section 940, and extends distally to a distal end 954. The length of the third section 950 may be in the range of about 1mm to about 5mm. The third section 950 includes a generally cylindrical outer surface 956 with the third outer diameter OD3 extending from the proximal end 952 to the distal end 954.

The fourth section 960 of the distal transition portion 920 includes a proximal end 962 and a distal end 964. The proximal end 962 is disposed adjacent to the distal end 954 of the third section 950 and extends distally to the distal end 964. The length of the fourth section 960 may be in the range of about 1mm to about 5mm. The fourth section 960 has the third outer diameter OD3 at the proximal end 962 and a fourth outer diameter OD4 at the distal end 964. The third outer diameter OD3 is smaller than the fourth outer diameter OD4. The fourth outer diameter OD4 may be in the range of about 2.75mm to about 11mm. In embodiments herein, an outer surface 966 of the fourth section 960 flares outwardly and distally in a concave profile from the third outer diameter OD3 at the proximal end 962 to the fourth outer diameter OD4 at the distal end 964.

The fifth section 970 of the distal transition portion 920 includes a proximal end 972 and a distal end 974. The proximal end 972 is disposed adjacent to the distal end 964 of the fourth section 960. The fifth section 970 extends distally from the proximal end 972 to the distal end 974. The distal end 974 is disposed adjacent to the proximal end of the distal cylindrical portion 722 of the distal portion 718 of the dilator 700. The length of the fifth section 970 may be in the range of about 1mm to about 5mm. The fifth section 970 has the fourth outer diameter OD4 at the proximal end 972 and a fifth outer diameter OD5 at the distal end 974, wherein the fourth outer diameter OD4 is smaller than the fifth outer diameter OD5. The fifth outer diameter OD5 may be in the range of about 3mm to about 12mm. In embodiments herein, an outer surface 976 of the fifth section 970 flares outwardly and distally in a convex profile from the fourth outer diameter OD4 at the proximal end 972 to the fifth outer diameter OD5 at the distal end 964. The embodiment of FIGS 55-56 may be described as a double taper (in the proximal direction) or a double flare (in the distal direction).

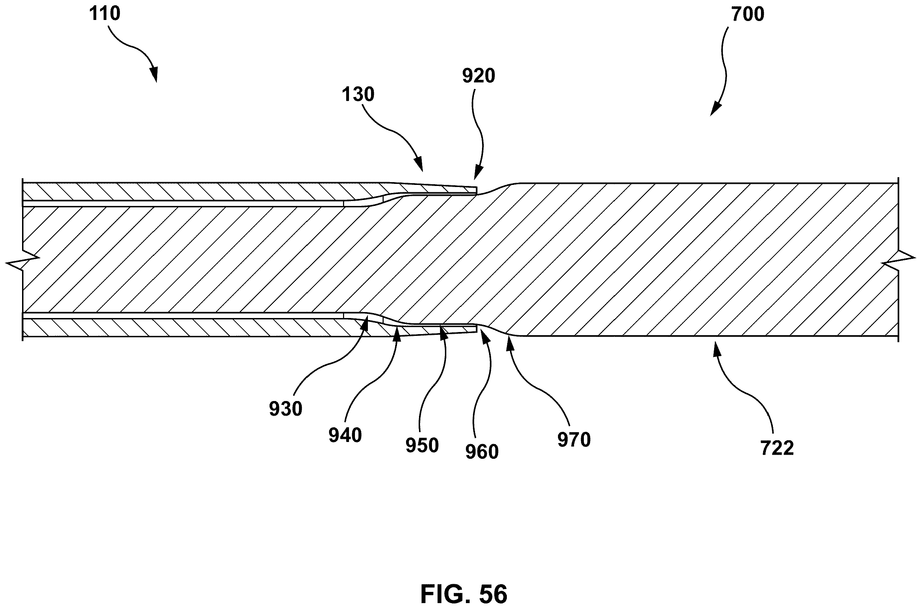

FIG. 56 shows a distal portion of the introducer sheath 110 disposed on an outer surface of the dilator 700, with the tip 130 of the introducer sheath 110 disposed on the distal transition portion 920 on first section 930, the second section 940, the third section 950, and a portion of the fourth section 960. In embodiments herein, utilizing the distal transition portion 920, the transition between the tip 130 and the distal cylindrical portion 722 of the dilator 700 is generally smooth or flat to provide a reduced likelihood of the tip 130 catching or snagging tissue within the vessel.

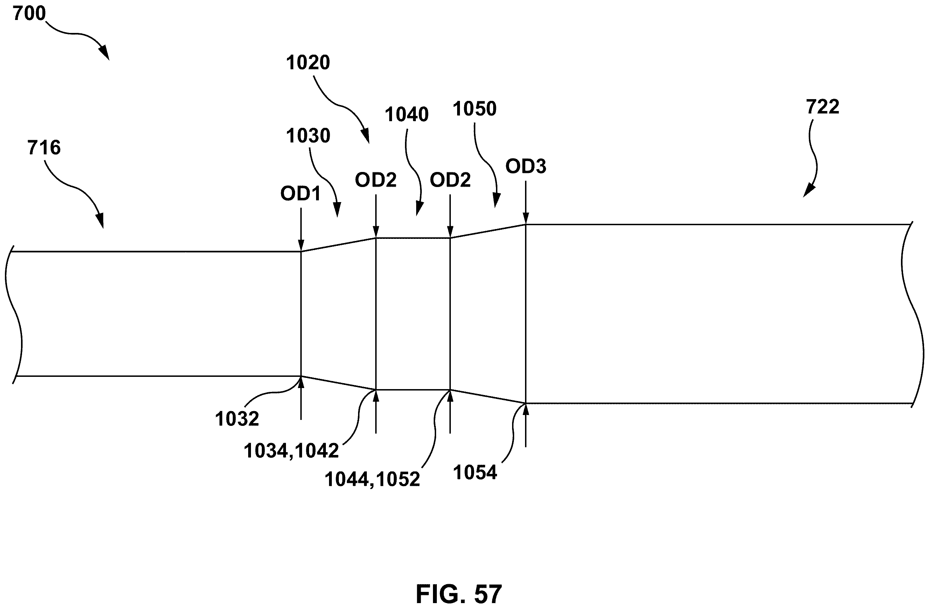

FIGS. 57-58 show a distal transition portion 1020 according to another embodiment hereof. The distal transition portion 1020 may be used with embodiments of the dilator 700 described above. As shown in FIG. 57, the distal transition portion 1020 includes a first or proximal section 1030, a second or middle section 1040, and a third or distal section 1050.

The first section 1030 includes a proximal end 1032 and a distal end 1034. The proximal end 1032 is disposed adjacent to the distal end of the middle portion 716 of the dilator 700. The first section 1030 extends distally from the proximal end 1032 to the distal end 1034. The length of the first section 1030 may be in the range of about 2mm to about 5mm. The first section 1030 has a first outer diameter OD1 at the proximal end 1032 and a second outer diameter OD2 at the distal end 1034. In embodiments herein, an outer surface 1036 of the first section 1030 is flared linearly in a direction towards the distal end 1034 such that the first outer diameter OD1 at the proximal end 1032 is smaller than the second outer diameter OD2 at the distal end 1034. The first outer diameter OD1 may be in the range of about 2mm to about 10mm. The second outer diameter OD2 may be in the range of about 2.5mm to about 11mm.

The second or middle section 1040 of the distal transition portion 1020 includes a proximal end 1042 disposed adjacent to the distal end 1034 of the first section 1030. The second section 1040 extends distally from the proximal end 1042 to a distal end 1044. The length of the second section 1040 may be in the range of about 1mm to about 5mm. The second section 1040 includes a generally cylindrical outer surface 1046 with the second outer diameter OD2 extending from the proximal end 1042 to the distal end 1044.

The third or distal section 1050 of the distal transition portion 1020 includes a proximal end 1052 and a distal end 1054. The proximal end 1052 is disposed adjacent to the distal end 1034 of the second section 1040 and extends distally to the distal end 1054. The distal end 1054 is disposed adjacent to the proximal end of the distal cylindrical portion 722 of the distal portion 718 of the dilator 700. The length of the third section 1050 may be in the range of about 1mm to about 5mm. The third section 1050 includes an outer surface 1056. The outer surface 1056 is flared linearly from the proximal end 1052 to the distal end 1054 such that the second outer diameter OD2 at the proximal end 1052 is smaller than a third outer diameter OD3 at the distal end 1054. The third outer diameter OD3 may be in the range of about 3mm to about 12mm.

The distal transition portion 1020 is similar to the distal transition portion 920 in that it includes a double taper or double flare. However, the distal transition portion 1020 includes linear flares instead of the double curved flares of the distal transition portion 920.

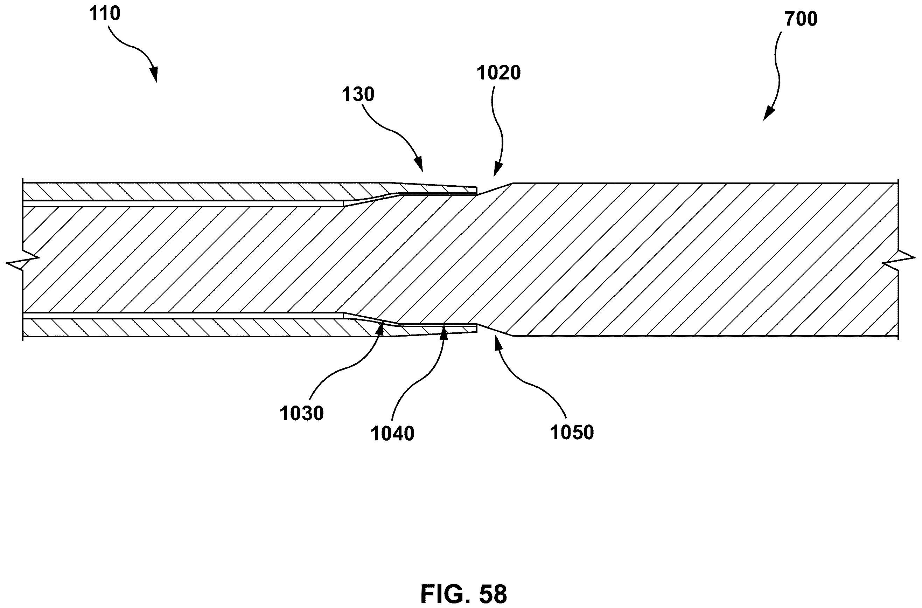

As shown in FIG. 58, a distal portion of the introducer sheath 110 is disposed on an outer surface of the dilator 700. In greater detail, the tip 130 of the introducer sheath 110 is disposed on the distal transition portion 1020 along first section 1030 and the second section 1040. As shown in FIG. 58, utilizing the distal transition portion 1020, the transition between the tip 130 and the distal cylindrical portion 722 of the dilator 700 is generally smooth or flat to provide a reduced likelihood of the tip 130 catching or snagging tissue within the vessel.

In embodiments herein, the distal transition portions 720, 820, 920, and 1020 may each be formed of materials such as, but not limited to PTFE, FEP, PeBax, HDPE, LDPE or any other suitable material. Further, embodiments herein may include a lubricious coating on an outer surface thereof and may contain radiopaque markers such that the dilator and sheath tip relationship may be monitored using standard imaging techniques.

As used herein, the terms "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye. The term "about" as used herein to refer to dimensions means within 5% of the dimension.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

The following examples are illustrative of the techniques described herein.

Example 1. An introducer comprising: an introducer hub, the introducer hub including a proximal end, a distal end, and a central lumen extending from the proximal end to the distal end, the introducer hub including a distal tube at a distal portion of the introducer hub, the distal tube defining a portion of the central lumen; a sheath coupled to the introducer hub and extending distally therefrom; and a connector coupling the sheath to the distal tube of the introducer hub.

Example 2. The introducer of Example 1, wherein: the distal tube comprises a cylinder having a stepped outer surface and including tube threads on a portion of the stepped outer surface; the connector comprises a cap comprising a cylinder having a stepped inner surface and including cap threads on a portion of the stepped inner surface; and the cap is configured to capture a proximal end of the sheath between the stepped inner surface of the cap and the stepped outer surface of the distal tube as the cap threads engage with the tube threads.

Example 3. The introducer of Example 2, wherein: the distal tube further comprises a ratchet lock proximal of the tube threads, the ratchet lock comprising a plurality of teeth extending radially outwardly and circumferentially around the distal tube; and the cap further comprises at least one tooth extending radially inwardly proximal of the cap threads, wherein the at least one tooth is configured to engage the plurality of teeth of the ratchet lock to lock the cap to the distal tube.

Example 4. The introducer of Example 2, wherein: the distal tube comprises a tube lip extending radially outwardly proximal of the tube threads; the cap includes a plurality of longitudinal legs extending proximally such that a proximal end of the legs is proximal of the cap threads, the plurality of longitudinal legs disposed adjacently around a circumference of the cap and including gaps between circumferentially adjacent longitudinal legs, the plurality of longitudinal legs comprising a cap lips extending radially inwards from the proximal end of the longitudinal legs; and the plurality of longitudinal legs are configured to flex radially outwardly for the cap lips to extend over the tube lip and are configured to flex back radially inwardly when the leg lips extend proximal of the tube lip such that the leg lips lock the cap to the distal tube.

Example 5. The introducer of Example 1, wherein the connector comprises a housing, a grab ring, and an O-ring, wherein the housing comprises a tube including distal lip extending radially inwardly from a distal end of the housing, a plurality of longitudinal legs at a proximal end of tube, the longitudinal legs separated circumferentially by a plurality of gaps between adjacent legs of plurality of longitudinal legs, and a proximal lip extending radially inward from a proximal end of the longitudinal legs; the distal tube includes a first groove extending radially inwardly from an outer surface of the distal tube and circumferentially around the distal tube and a second groove extending radially outwardly from an inner surface of the distal tube and circumferentially around the inner surface of the distal tube; and with a proximal end of the sheath disposed within the distal tube; the O-ring is disposed in the second groove of the distal tube between an inner surface of the distal tube and an outer surface of the sheath; and the housing is configured to be translated over the sheath and over the distal tube proximally such that: the longitudinal legs flex radially outwardly as the proximal lip extend over the distal tube until the proximal lip reaches the first groove such that the longitudinal legs flex back radially inwardly as the proximal lip is disposed within the first groove; and the distal lip of the housing extends radially inwardly distal of the distal end of the distal tube and abuts the outer surface of the sheath.

Example 6. The introducer of Example 1, wherein: the connector comprises a locking clip configured to be disposed over the sheath, wherein the locking clip comprises a generally cylindrical tube including at least one clip arm proximal of a distal end of the locking clip, wherein the at least one clip arm is configured to flex radially inwardly and outwardly and includes a clip lip extending radially outwardly from the clip arm; the distal tube includes at least one slot extending from an outer surface to an inner surface thereof; the sheath includes a proximal lip extending radially outwardly therefrom; and with the locking clip disposed over the sheath such that a proximal end of the locking clip is adjacent the proximal lip of the sheath, the sheath and locking clip are configured to be inserted into the distal tube such that the at least one clip arm flexes radially inwardly until the clip lip aligns with the slot of the distal tube such the at least one clip arm flexes back radially outwardly and the clip lip extends into the slot to couple the sheath and the locking clip to the distal tube.

Example 7. The introducer of Example 1, wherein: the connector comprises a generally cylindrical tube including a distal portion overmolded over a proximal end of the sheath, a proximal portion configured to be disposed over the distal tube of the introducer hub, and band configured to be disposed around the proximal portion to couple the proximal portion to the distal tube of the introducer hub; the distal tube of the introducer hub includes barbs extending radially outwardly along the distal tube, wherein the band of the connector is configured to deform the proximal portion of the generally cylindrical tube of the connector to couple the generally cylindrical tube of the connector to the distal tube of the introducer hub; and each barb extends circumferentially around the distal tube, and wherein each barb includes a barb distal end, a ramp extending radially outwardly and proximally from the barb distal end to a barb proximal end, and a shoulder at the barb proximal end extending radially inwardly form the barb proximal end.

Example 8. A system comprising: a dilator comprising a dilator hub and a dilator shaft coupled to and extending distally from the dilator hub, the dilator shaft including: a proximal portion having a first outer diameter; a middle portion having a second outer diameter; a proximal transition portion disposed between the proximal portion and the middle portion, the proximal transition portion tapering in a distal direction from the first outer diameter to the second outer diameter; and a distal portion extending distally from the middle portion, the distal portion including a distal cylindrical portion having a third outer diameter, wherein the first outer diameter is larger than the second outer diameter and the third outer diameter, and wherein the third outer diameter is larger than the second outer diameter.

Example 9. The system of Example 8, wherein the dilator shaft further includes a distal transition portion disposed between the middle portion and the distal cylindrical portion, wherein the distal transition portion flares outwardly distally from the second outer diameter of the middle portion to the third outer diameter of the distal cylindrical portion.

Example 10. The system of Example 9, wherein the distal transition portion flares linearly outwardly from a proximal end adjacent a distal end of the middle portion to a distal end of the distal transition portion adjacent a proximal end of the distal cylindrical portion.

Example 11. The system of Example 9, wherein the distal transition portion includes a proximal section and a distal section, the proximal section flaring radially outwardly with a curved concave profile, and a distal section flaring outwardly with a curved convex profile.

Example 12. The system of Example 9, wherein the distal transition portion includes a proximal section extending distally from the middle portion, a middle section extending distally from the proximal section, and a distal section distal extending distally from the middle section, wherein the proximal section flares outwardly in a distal direction, the middle section is generally cylindrical, and the distal section flares outwardly in a distal direction.

Example 13. The system of Example 12, wherein the proximal section and the distal section flare linearly outwardly.

Example 14. The system of Example 12, wherein each of the proximal section and the distal section includes curved concave profile and a curved convex profile.

Example 15. The system of Example 8, wherein the distal portion of the dilator shaft further includes a distal tapered tip extending distally from the distal cylindrical portion wherein the distal tapered tip includes an outer diameter than tapers distally.

Example 16. The system of Example 8, wherein the system further comprises an introducer comprising an introducer hub and an expandable sheath coupled to and extending distally from the introducer hub, wherein the expandable sheath is expandable from a radially compressed state wherein the expandable sheath has a first inner sheath diameter and a first outer sheath diameter to a radially expanded state wherein the sheath has a second inner sheath diameter larger than the first inner sheath diameter and a second outer sheath diameter larger than the first outer sheath diameter.

Example 17. The system of Example 16, wherein: the first outer diameter of the dilator shaft proximal portion is sized to fit within the introducer hub; the second outer diameter of the dilator shaft middle portion is smaller than the first inner sheath diameter of the sheath; and the third outer diameter of the dilator shaft distal cylindrical portion is approximately equal to the first outer sheath diameter of the sheath.

Example 18. An expandable introducer sheath comprising: a shaft configured to be expanded from a radially unexpanded, folded state to a radially expanded, unfolded state in reaction to a device being extended through a central lumen of the expandable sheath, wherein the shaft includes: an inner liner; and an outer wall disposed radially outwardly of the inner liner, the outer wall having a variable thickness around a circumference of the shaft; and a tip coupled to a distal portion of the shaft, the tip including a first circumferential portion and a second circumferential portion, wherein the second circumferential portion is more flexible than the first circumferential such that the second circumferential portion is configured to radially expand when the in reaction to the device extending through tip.

Example 19. The expandable introducer sheath of Example 18, wherein in the radially expanded, unfolded state, the shaft includes a thick-walled region and a thin-walled region, wherein the thin-walled region is configured to fold when the shaft is in the radially unexpanded, folded state.

Example 20. The expandable introducer sheath of Example 18, wherein in the radially unexpanded, folded state, the shaft includes a folded region and an unfolded region, wherein the second circumferential portion is circumferentially aligned with the folded region.

## Claims

1. An introducer comprising:
an introducer hub, the introducer hub including a proximal end, a distal end, and a central lumen extending from the proximal end to the distal end, the introducer hub including a distal tube at a distal portion of the introducer hub, the distal tube defining a portion of the central lumen;
a sheath coupled to the introducer hub and extending distally therefrom; and
a connector coupling the sheath to the distal tube of the introducer hub.

2. The introducer of claim 1, wherein:
the distal tube comprises a cylinder having a stepped outer surface and including tube threads on a portion of the stepped outer surface;
the connector comprises a cap comprising a cylinder having a stepped inner surface and including cap threads on a portion of the stepped inner surface; and
the cap is configured to capture a proximal end of the sheath between the stepped inner surface of the cap and the stepped outer surface of the distal tube as the cap threads engage with the tube threads.

3. The introducer of claim 2, wherein:
the distal tube further comprises a ratchet lock proximal of the tube threads, the ratchet lock comprising a plurality of teeth extending radially outwardly and circumferentially around the distal tube; and
the cap further comprises at least one tooth extending radially inwardly proximal of the cap threads, wherein the at least one tooth is configured to engage the plurality of teeth of the ratchet lock to lock the cap to the distal tube.

4. The introducer of claim 2, wherein:
the distal tube comprises a tube lip extending radially outwardly proximal of the tube threads;
the cap includes a plurality of longitudinal legs extending proximally such that a proximal end of the legs is proximal of the cap threads, the plurality of longitudinal legs disposed adjacently around a circumference of the cap and including gaps between circumferentially adjacent longitudinal legs, the plurality of longitudinal legs comprising a cap lips extending radially inwards from the proximal end of the longitudinal legs; and
the plurality of longitudinal legs are configured to flex radially outwardly for the cap lips to extend over the tube lip and are configured to flex back radially inwardly when the leg lips extend proximal of the tube lip such that the leg lips lock the cap to the distal tube.

5. The introducer of claim 1, wherein the connector comprises a housing, a grab ring, and an O-ring, wherein
the housing comprises a tube including distal lip extending radially inwardly from a distal end of the housing, a plurality of longitudinal legs at a proximal end of tube, the longitudinal legs separated circumferentially by a plurality of gaps between adjacent legs of plurality of longitudinal legs, and a proximal lip extending radially inward from a proximal end of the longitudinal legs;
the distal tube includes a first groove extending radially inwardly from an outer surface of the distal tube and circumferentially around the distal tube and a second groove extending radially outwardly from an inner surface of the distal tube and circumferentially around the inner surface of the distal tube; and
with a proximal end of the sheath disposed within the distal tube;
the O-ring is disposed in the second groove of the distal tube between an inner surface of the distal tube and an outer surface of the sheath; and
the housing is configured to be translated over the sheath and over the distal tube proximally such that:
the longitudinal legs flex radially outwardly as the proximal lip extend over the distal tube until the proximal lip reaches the first groove such that the longitudinal legs flex back radially inwardly as the proximal lip is disposed within the first groove; and
the distal lip of the housing extends radially inwardly distal of the distal end of the distal tube and abuts the outer surface of the sheath.

6. The introducer of claim 1, wherein:
the connector comprises a locking clip configured to be disposed over the sheath, wherein the locking clip comprises a generally cylindrical tube including at least one clip arm proximal of a distal end of the locking clip, wherein the at least one clip arm is configured to flex radially inwardly and outwardly and includes a clip lip extending radially outwardly from the clip arm;
the distal tube includes at least one slot extending from an outer surface to an inner surface thereof;
the sheath includes a proximal lip extending radially outwardly therefrom; and
with the locking clip disposed over the sheath such that a proximal end of the locking clip is adjacent the proximal lip of the sheath, the sheath and locking clip are configured to be inserted into the distal tube such that the at least one clip arm flexes radially inwardly until the clip lip aligns with the slot of the distal tube such the at least one clip arm flexes back radially outwardly and the clip lip extends into the slot to couple the sheath and the locking clip to the distal tube.

7. The introducer of claim 1, wherein:
the connector comprises a generally cylindrical tube including a distal portion overmolded over a proximal end of the sheath, a proximal portion configured to be disposed over the distal tube of the introducer hub, and band configured to be disposed around the proximal portion to couple the proximal portion to the distal tube of the introducer hub;
the distal tube of the introducer hub includes barbs extending radially outwardly along the distal tube, wherein the band of the connector is configured to deform the proximal portion of the generally cylindrical tube of the connector to couple the generally cylindrical tube of the connector to the distal tube of the introducer hub; and
each barb extends circumferentially around the distal tube, and wherein each barb includes a barb distal end, a ramp extending radially outwardly and proximally from the barb distal end to a barb proximal end, and a shoulder at the barb proximal end extending radially inwardly form the barb proximal end.

8. A system comprising:
a dilator comprising a dilator hub and a dilator shaft coupled to and extending distally from the dilator hub, the dilator shaft including:
a proximal portion having a first outer diameter;
a middle portion having a second outer diameter;
a proximal transition portion disposed between the proximal portion and the middle portion, the proximal transition portion tapering in a distal direction from the first outer diameter to the second outer diameter; and
a distal portion extending distally from the middle portion, the distal portion including a distal cylindrical portion having a third outer diameter,
wherein the first outer diameter is larger than the second outer diameter and the third outer diameter, and
wherein the third outer diameter is larger than the second outer diameter.

9. The system of claim 8, wherein the dilator shaft further includes a distal transition portion disposed between the middle portion and the distal cylindrical portion, wherein the distal transition portion flares outwardly distally from the second outer diameter of the middle portion to the third outer diameter of the distal cylindrical portion.

10. The system of claim 9, wherein:
the distal transition portion flares linearly outwardly from a proximal end adjacent a distal end of the middle portion to a distal end of the distal transition portion adjacent a proximal end of the distal cylindrical portion; or
the distal transition portion includes a proximal section and a distal section, the proximal section flaring radially outwardly with a curved concave profile, and a distal section flaring outwardly with a curved convex profile; or
the distal transition portion includes a proximal section extending distally from the middle portion, a middle section extending distally from the proximal section, and a distal section distal extending distally from the middle section, wherein the proximal section flares outwardly in a distal direction, the middle section is generally cylindrical, and the distal section flares outwardly in a distal direction.

11. The system of any one of claims 8 to 10, wherein:
the proximal section and the distal section flare linearly outwardly; or
each of the proximal section and the distal section includes a curved concave profile and a curved convex profile.

12. The system of any one of claims 8 to 11, wherein the distal portion of the dilator shaft further includes a distal tapered tip extending distally from the distal cylindrical portion wherein the distal tapered tip includes an outer diameter than tapers distally.

13. The system of any one of claims 8 to 12, further comprising:
an introducer of any one of claims 1 to 7.

14. The system of claim 13, wherein:
the first outer diameter of the dilator shaft proximal portion is sized to fit within the introducer hub;
the second outer diameter of the dilator shaft middle portion is smaller than the first inner sheath diameter of the sheath; and
the third outer diameter of the dilator shaft distal cylindrical portion is approximately equal to the first outer sheath diameter of the sheath.
